# EUROPÄISCHE PATENTSCHRIFT

(11) **EP 3 110 796 B1**
(45) Veröffentlichungstag und Bekanntmachung des Hinweises auf die Patenterteilung: **06.12.2017**
(21) Anmeldenummer: 15704709.3
(22) Anmeldetag: 27.01.2015
(51) Int. Cl.: C07D 209/00, C07D 333/76, C09K 11/06, C07D 403/10, H01L 51/00, C07C 13/00, C07D 471/16, C07D 471/20, C07D 471/22, C07D 487/04, C07D 491/147, C07D 495/20, C07D 498/04, C07D 498/10, C07D 513/04, C07D 513/10, C07D 519/00, C07D 209/96, C09K 11/02, H01L 51/50

(54) **MATERIALIEN FÜR ORGANISCHE ELEKTROLUMINESZENZVORRICHTUNGEN**
MATERIALS FOR ORGANIC LIGHT-EMITTING DEVICES
MATIÈRES POUR DISPOSITIFS ÉLECTROLUMINESCENTS ORGANIQUES

(30) Priorität: 28.02.2014 EP 14000729
(43) Veröffentlichungstag der Anmeldung: 04.01.2017
(73) Patentinhaber: Merck Patent GmbH, 64293 Darmstadt (DE)
(72) Erfinder: PARHAM, Amir Hossain, 60486 Frankfurt am Main (DE); JATSCH, Anja, 60489 Frankfurt am Main (DE); GROSSMANN, Tobias, 64297 Darmstadt (DE); EBERLE, Thomas, 76829 Landau (DE); KROEBER, Jonas Valentin, 60311 Frankfurt am Main (DE); LINGE, Rouven, 64291 Darmstadt (DE)
(86) Internationale Anmeldenummer: PCT/EP2015/000147
(87) Internationale Veröffentlichungsnummer: WO 2015/128052

(56) Entgegenhaltungen:
- WO-A1-2006/122630
- WO-A1-2007/043354
- WO-A1-2012/008557
- WO-A1-2013/064206
- WO-A1-2014/017042
- CN-A- 102 229 565
- DE-A1-102010 019 306

## Beschreibung

Die vorliegende Erfindung betrifft Materialien für die Verwendung in elektronischen Vorrichtungen, insbesondere als Hostmaterial für phosphoreszierende Emitter in organischen Elektrolumineszenzvorrichtungen, sowie elektronische Vorrichtungen, insbesondere organische Elektrolumineszenzvorrichtungen, enthaltend diese Materialien.

In organischen Elektrolumineszenzvorrichtungen (OLEDs) werden als emittierende Materialien zunehmend metallorganische Komplexe eingesetzt, die Phosphoreszenz statt Fluoreszenz zeigen, insbesondere Iridium- oder Platinkomplexe. Aus quantenmechanischen Gründen ist unter Verwendung phosphoreszierender metallorganischer Verbindungen als Phosphoreszenzemitter eine bis zu vierfache Energie- und Leistungseffizienz möglich. Generell gibt es bei OLEDs, insbesondere auch bei OLEDs, die Triplettemission (Phosphoreszenz) zeigen, jedoch immer noch Verbesserungsbedarf, beispielsweise im Hinblick auf Effizienz, Betriebsspannung und Lebensdauer.

Die Eigenschaften von phosphoreszierenden OLEDs werden nicht nur von den eingesetzten Triplettemittern bestimmt. Hier sind insbesondere auch die anderen verwendeten Materialien, wie Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können somit auch zu deutlichen Verbesserungen der OLED-Eigenschaften führen. Auch für fluoreszierende OLEDs gibt es bei diesen Materialien noch Verbesserungsbedarf.

Gemäß dem Stand der Technik werden unter anderem Lactame, z. B. gemäß WO 2011/137951 oder WO 2013/064206, als Matrixmaterialien für phosphoreszierende Emitter verwendet. Allerdings besteht bei Verwendung dieser Matrixmaterialien ebenso wie bei anderen Matrixmaterialien noch Verbesserungsbedarf, insbesondere in Bezug auf die Effizienz und die Lebensdauer der Vorrichtung.

Aufgabe der vorliegenden Erfindung ist die Bereitstellung von Verbindungen, welche sich für den Einsatz in einer fluoreszierenden oder insbesondere in einer phosphoreszierenden OLED eignen, beispielsweise als Matrixmaterial oder als Elektronentransport- bzw. Lochblockiermaterial, je nach genauem Substitutionsmuster. Insbesondere ist es die Aufgabe der vorliegenden Erfindung, Matrixmaterialien bereitzustellen, welche sich für grün, gelb und rot phosphoreszierende OLEDs eignen.

Überraschend wurde gefunden, dass die unten näher beschriebenen Verbindungen diese Aufgabe lösen und zu deutlichen Verbesserungen der organischen Elektrolumineszenzvorrichtung führen, insbesondere hinsichtlich der Lebensdauer, der Effizienz und der Betriebsspannung. Dies gilt insbesondere für rot, gelb und grün phosphoreszierende Elektrolumineszenzvorrichtungen, vor allem bei Einsatz der Verbindungen als Matrixmaterial. Die Materialien zeichnen sich weiterhin durch eine hohe Temperaturstabilität aus. Diese Verbindungen sowie elektronische Vorrichtungen, insbesondere organische Elektrolumineszenzvorrichtungen, welche diese Verbindungen enthalten, sind daher der Gegenstand der vorliegenden Erfindung.

Gegenstand der vorliegenden Erfindung ist eine Verbindung gemäß der folgenden Formel (1), 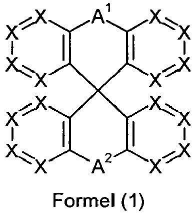 wobei für die verwendeten Symbole gilt:
- X: ist bei jedem Auftreten gleich oder verschieden CR oder N oder zwei benachbarte Gruppen X stehen zusammen für O, S oder NR, so dass sich ein Fünfring ergibt, oder zwei benachbarte Gruppen X stehen zusammen für eine Gruppe der folgenden Formel (2), (3) oder (4),
mit der Maßgabe, dass die Verbindung der Formel (1) mindestens eine Gruppe der Formel (2) aufweist, 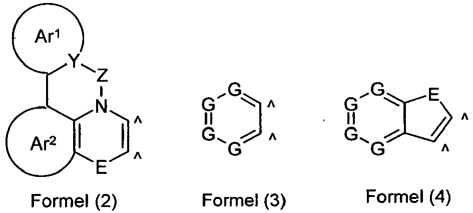 wobei ^ die entsprechenden benachbarten Gruppen X in Formel (1) kennzeichnet, d. h. an diesen Positionen ist die Gruppe der Formel (2) bzw. (3) bzw. (4) an die Verbindung der Formel (1) ankondensiert;
- A¹, A²: ist gleich oder verschieden bei jedem Auftreten eine Einfachbindung, CR₂, NR, O, S oder C=O;
- Z: ist gleich oder verschieden bei jedem Auftreten C=O, C=S, CR₂, BR, SiR₂, P(=O)R, SO oder SO₂;
- Y: ist C, wenn Ar¹ eine 6-Ring-Aryl- oder Heteroarylgruppe darstellt, bzw. ist C oder N, wenn Ar¹ eine 5-Ring-Heteroarylgruppe darstellt;
- E: ist gleich oder verschieden bei jedem Auftreten eine Einfachbindung, CR₂, NR, O, S oder C=O, mit der Maßgabe, dass E in Formel (4) keine Einfachbindung darstellt;
- Ar¹: ist gleich oder verschieden bei jedem Auftreten zusammen mit der Gruppe Y und dem explizit dargestellten Kohlenstoffatom ein aromatisches oder heteroaromatisches Ringsystem mit 5 bis 24 aromatischen Ringatomen, welches durch einen oder mehrere Reste R substituiert sein kann;
- Ar²: ist gleich oder verschieden bei jedem Auftreten zusammen mit den drei explizit dargestellten Kohlenstoffatomen ein aromatisches oder heteroaromatisches Ringsystem mit 5 bis 24 aromatischen Ringatomen, welches durch einen oder mehrere Reste R substituiert sein kann;
- G: ist gleich oder verschieden bei jedem Auftreten CR oder N;
- R: ist bei jedem Auftreten gleich oder verschieden ausgewählt aus der Gruppe bestehend aus H, D, F, Cl, Br, I, CN, NO₂, N(Ar³)₂, N(R¹)₂, C(=O)Ar³, C(=O)R¹, P(=O)(Ar³)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R¹ substituiert sein kann, wobei eine oder mehrere nicht-benachbarte CH₂-Gruppen durch R¹C=CR¹, C≡C, Si(R¹)₂, C=O, C=NR¹, P(=O)(R¹), SO, SO₂, NR¹, O, S oder CONR¹ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R¹ substituiert sein kann, einer Aryloxy- oder Heteroaryloxygruppe mit 5 bis 40 aromatischen Ringatomen, die mit einem oder mehreren Resten R¹ substituiert sein kann, oder einer Aralkyl- oder Heteroaralkylgruppe mit 5 bis 40 aromatischen Ringatomen, die mit einem oder mehreren Resten R¹ substituiert sein kann, wobei optional zwei oder mehr benachbarte Substituenten R ein monocyclisches oder polycyclisches, aliphatisches, aromatisches oder heteroaromatisches Ringsystem bilden können, das mit einem oder mehreren Resten R¹ substituiert sein kann;
- Ar³: ist bei jedem Auftreten gleich oder verschieden ein aromatisches oder heteroaromatisches Ringsystem mit 5-30 aromatischen Ringatomen, das mit einem oder mehreren nicht-aromatischen Resten R¹ substituiert sein kann; dabei können zwei Reste Ar³, welche an dasselbe N-Atom oder P-Atom binden, auch durch eine Einfachbindung oder eine Brücke, ausgewählt aus N(R¹), C(R¹)₂ oder O, miteinander verbrückt sein;
- R¹: ist bei jedem Auftreten gleich oder verschieden ausgewählt aus der Gruppe bestehend aus H, D, F, Cl, Br, I, CN, NO₂, N(R²)₂, C(=O)R², P(=O)(R²)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R² substituiert sein kann, wobei eine oder mehrere nicht-benachbarte CH₂-Gruppen durch R²C=CR², C=C, Si(R²)₂, C=O, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R² substituiert sein kann, einer Aryloxy- oder Heteroaryloxygruppe mit 5 bis 40 aromatischen Ringatomen, die mit einem oder mehreren Resten R² substituiert sein kann, oder einer Aralkyl- oder Heteroaralkylgruppe mit 5 bis 40 aromatischen Ringatomen, die mit einem oder mehreren Resten R² substituiert sein kann, wobei optional zwei oder mehr benachbarte Substituenten R¹ ein monocyclisches oder polycyclisches, aliphatisches, aromatisches oder heteroaromatisches Ringsystem bilden können, das mit einem oder mehreren Resten R² substituiert sein kann;
- R²: ist ausgewählt aus der Gruppe bestehend aus H, D, F, CN, einem aliphatischem Kohlenwasserstoffrest mit 1 bis 20 C-Atomen, einem aromatischem oder heteroaromatischem Ringsystem mit 5 bis 30 aromatischen Ringatomen, wobei zwei oder mehr benachbarte Substituenten R² miteinander ein mono- oder polycyclisches, aliphatisches, aromatisches oder heteroaromatisches Ringsystem bilden können.

Benachbarte Gruppen X im Sinne der vorliegenden Erfindung sind Gruppen X, die direkt aneinander gebunden sind. Benachbarte Substituenten im Sinne der vorliegenden Erfindung sind Substituenten, die an Atome gebunden, welche wiederum direkt aneinander gebunden sind, oder die an dasselbe Atom gebunden sind.

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 80 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 80 aromatischen Ringatomen, welches noch jeweils mit den oben genannten Resten R¹ oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

Jeder Cyclus mit Gruppen X in der Struktur der Formel (1) ist ein Fünf- oder ein Sechsring, wobei durch Ringbildung der Reste R auch kondensierte Strukturen entstehen können. Auch wenn benachbarte Gruppen X für eine Gruppe der Formel (2), (3) oder (4) stehen, entstehen größere kondensierte Strukturen. Dies bedeutet, dass maximal eine Gruppierung X-X pro Cyclus für O, S oder NR steht. Dabei ist es bevorzugt, wenn die Gruppe der Formel (2) in einem Sechsring gebunden ist. Weiterhin ist es bevorzugt, wenn jeder Cyclus maximal eine Gruppe der Formel (2), (3) oder (4) enthält. Wenn ein Cyclus in Formel (1) also eine Gruppe der Formel (2) enthält, ist es bevorzugt, wenn die anderen Gruppen X in diesem Cyclus gleich oder verschieden für CR oder N stehen, insbesondere für CR.

Es ist weiterhin bevorzugt, wenn pro Cyclus maximal eine Gruppe X für N steht. Besonders bevorzugt steht keine Gruppe X für N, d. h. alle Gruppen X, die nicht für eine Gruppe der Formel (2), (3) oder (4) oder für O, S oder NR stehen, stehen besonders bevorzugt für CR.

Wenn Gruppen der Formel (3) oder (4) vorhanden sind, so ist es bevorzugt, wenn maximal eine Gruppe G in diesen Formeln für N steht. Besonders bevorzugt stehen in den Gruppen der Formel (3) und (4) alle Gruppen G für CR.

Im Folgenden ist schematisch erläutert, was mit der Bezeichnung "Cyclus" bzw. "Cyclus mit den Gruppen X" in Formel (1) und in der Definition von X gemeint ist:

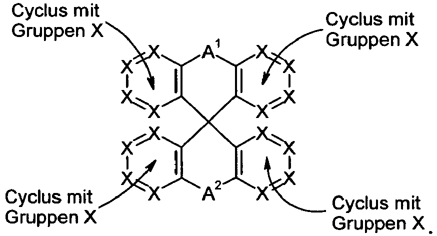

In einer bevorzugten Ausführungsform der Erfindung steht mindestens eine der Gruppen A¹ und A² für eine Einfachbindung. In diesem Fall ist es bevorzugt, wenn eine Gruppe der Formel (2) auf derselben Hälfte der Spiroverbindung gebunden ist, auf der auch diese Gruppe A¹ bzw. A², die für eine Einfachbindung steht, vorliegt. In einer besonders bevorzugten Ausführungsform der Erfindung stehen A¹ und A² für eine Einfachbindung.

Erfindungsgemäß stehen in mindestens einem der Cyclen zwei benachbarte Gruppen X für eine Gruppe der Formel (2). In einer bevorzugten Ausführungsform der Erfindung enthält die Verbindung der Formel (1) eine oder zwei Gruppen der Formel (2), besonders bevorzugt genau eine Gruppe der Formel (2).

Dabei können die Gruppen der Formel (2) in jeder Position und in jeder Orientierung gebunden sein. Geeignete Ausführungsformen der Formel (1), die genau eine Gruppe der Formel (2) aufinieisen, sind daher die Verbindungen der Formel (5) bis (10), 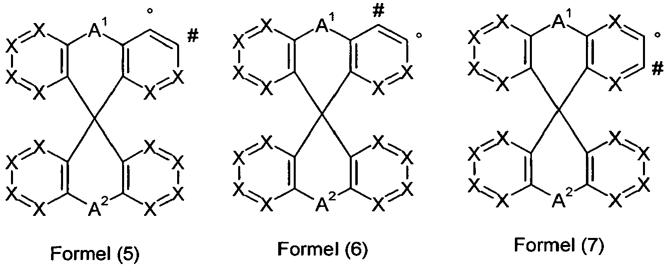 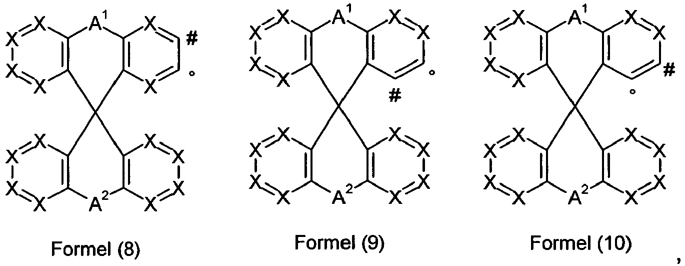 wobei ° die Verknüpfung mit dem Stickstoffatom der Gruppe der Formel (2) darstellt, # die Verknüpfung mit der Gruppe E der Gruppe der Formel (2) darstellt und die weiteren verwendeten Symbole die oben genannten Bedeutungen aufweisen. Dabei stehen bevorzugt die beiden Gruppen X in dem Cyclus, an dem die Gruppe der Formel (2) gebunden ist, für CR oder N, insbesondere für CR.

Wenn die Verbindung der Formel (1) zwei Gruppen der Formel (2) aufweist, so können diese Gruppen entweder an derselben Hälfte der Spiroverbindung gebunden sein, wie in der folgenden Formel (11) schematisch angedeutet, oder sie können an den beiden unterschiedlichen Hälften der Spiroverbindung gebunden sein, wie in der folgenden Formel (12) schematisch angedeutet,

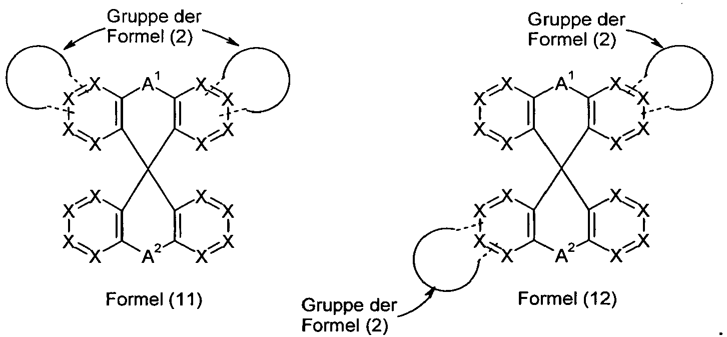

Dabei können die Gruppen der Formel (2) wiederum in allen Positionen und allen möglichen Orientierungen gebunden sein. Eine bevorzugte Ausführungsform für Verbindungen mit zwei Gruppen der Formel (2) ist die Verbindung der folgenden Formel (12a), 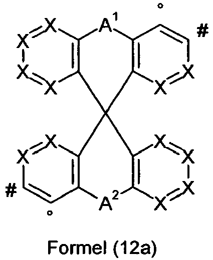 wobei die verwendeten Symbole die oben genannten Bedeutungen aufweisen.

Bevorzugt sind Verbindungen der Formel (5) bis (10), in denen mindestens eine der Gruppen A¹ und A² für eine Einfachbindung steht. Besonders bevorzugt sind Verbindungen, in denen A¹ und A² jeweils für eine Einfachbindung stehen.

Bevorzugt sind weiterhin Verbindungen der Formel (5) bis (10), in denen alle Gruppen X gleich oder verschieden bei jedem Auftreten für CR stehen.

Ganz besonders bevorzugt sind Verbindungen, in denen A¹ und A² jeweils für eine Einfachbindung steht und in denen alle Gruppen X gleich oder verschieden bei jedem Auftreten für CR stehen gemäß den folgenden Formeln (5a) bis (10a), 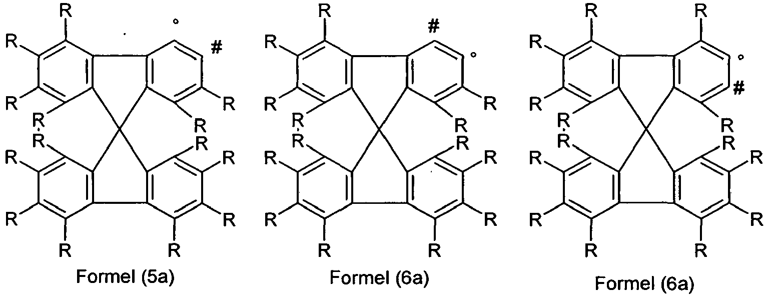 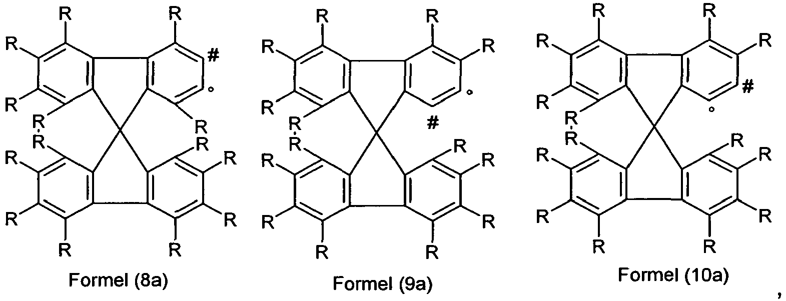 wobei die verwendeten Symbole die oben genannten Bedeutungen aufweisen. Besonders bevorzugt sind die Strukturen der Formeln (5a) und (8a).

Bevorzugte Verbindungen der Formeln (5a) bis (10a) sind die Verbindungen der Formeln (5b) bis (10b), 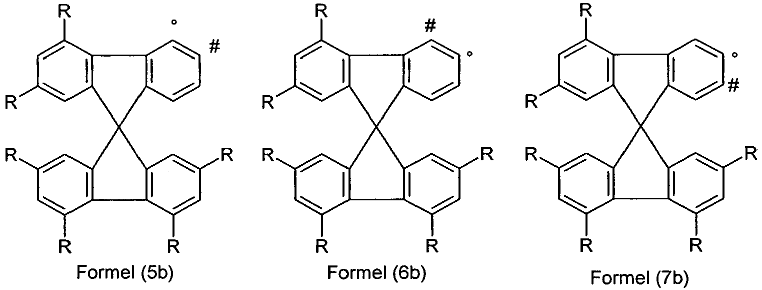 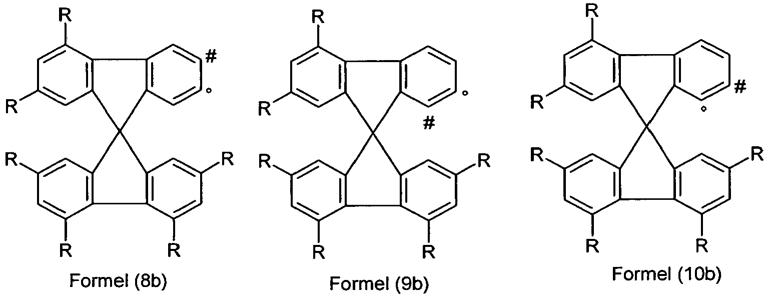 wobei die verwendeten Symbole die oben genannten Bedeutungen aufweisen.

Im Folgenden werden die Gruppen der Formel (2) beschrieben.

In einer bevorzugten Ausführungsform der Erfindung steht Z gleich oder verschieden für C=O oder C=S, besonders bevorzugt für C=O.

In einer weiteren bevorzugten Ausführungsform der Erfindung steht E für eine Einfachbindung, CR₂, C=O oder NR, besonders bevorzugt für eine Einfachbindung, CR₂ oder C=O und ganz besonders bevorzugt für eine Einfachbindung.

In einer weiteren bevorzugten Ausführungsform der Erfindung steht die Gruppe Ar¹ für eine Gruppe der folgenden Formel (13), (14), (15), (16) oder (17), 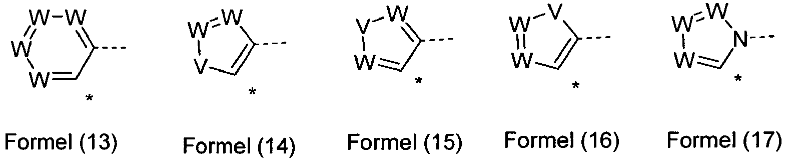 wobei die gestrichelte Bindung die Verknüpfung mit Z andeutet, * die Position der Verknüpfung mit Ar² andeutet und weiterhin gilt:
- W: ist gleich oder verschieden bei jedem Auftreten CR oder N; oder zwei benachbarte Gruppen W stehen für eine Gruppe der folgenden Formel (18) oder (19), 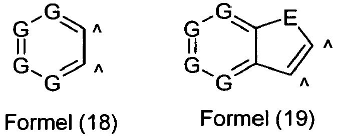 wobei E die oben genannte Bedeutung aufweist, jedoch bevorzugt keine Einfachbindung darstellt, G gleich oder verschieden bei jedem Auftreten für CR oder N steht und ^ die entsprechenden benachbarten Gruppen W in der Formel (13) bis (17) andeuten;
- V: ist NR, O oder S.

In einer weiteren bevorzugten Ausführungsform der Erfindung steht die Gruppe Ar² für eine Gruppe gemäß einer der folgenden Formeln (20), (21) oder (22), 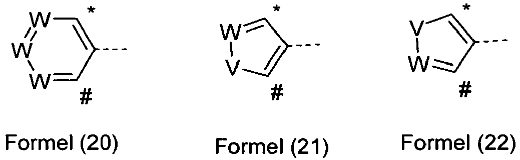 wobei die gestrichelte Bindung die Verknüpfung mit N andeutet, # die Position der Verknüpfung mit E andeutet, * die Verknüpfung mit Ar¹ andeutet und W und V die oben genannten Bedeutungen aufweisen.

In einer besonders bevorzugten Ausführungsform der Erfindung treten die oben genannten Bevorzugungen gleichzeitig auf. Besonders bevorzugt sind daher Gruppen gemäß Formel (2), für die gilt:
- Z: ist C=O oder C=S;
- E: ist gleich oder verschieden bei jedem Auftreten eine Einfachbindung, CR₂, C=O oder NR;
- Ar¹: ist ausgewählt aus den Gruppen der oben genannten Formeln (13), (14), (15), (16) oder (17);
- Ar²: ist ausgewählt aus den Gruppen der oben genannten Formeln (20), (21) oder (22).

In einer ganz besonders bevorzugten Ausführungsform der Erfindung gilt für Gruppen der Formel (2):
- Z: ist C=O;
- E: ist eine Einfachbindung;
- Ar¹: ist ausgewählt aus den Gruppen der oben genannten Formeln (13), (14), (15), (16) oder (17);
- Ar²: ist ausgewählt aus den Gruppen der oben genannten Formeln (20), (21) oder (22).

Dabei können die oben genannten bevorzugten Gruppen Ar¹ und Ar² beliebig miteinander kombiniert werden. Geeignete Kombinationen sind also die folgenden:

| Ar¹ | Ar² |
|---|---|
| Formel (13) | Formel (20) |
| Formel (13) | Formel (21) |
| Formel (13) | Formel (22) |
| Formel (14) | Formel (20) |
| Formel (14) | Formel (21) |
| Formel (14) | Formel (22) |
| Formel (15) | Formel (20) |
| Formel (15) | Formel (21) |
| Formel (15) | Formel (22) |
| Formel (16) | Formel (20) |
| Formel (16) | Formel (21) |
| Formel (16) | Formel (22) |
| Formel (17) | Formel (20) |
| Formel (17) | Formel (21) |
| Formel (17) | Formel (22) |

Besonders bevorzugt steht mindestens eine der Gruppen Ar¹ und Ar² für eine 6-Ring-Aryl- oder eine 6-Ring-Heteroarylgruppe. Besonders bevorzugt stehen beide Gruppen Ar¹ und Ar² für eine 6-Ring-Aryl- oder eine 6-Ring-Heteroarylgruppe. Besonders bevorzugt steht also Ar¹ für eine Gruppe der Formel (13) und gleichzeitig steht Ar² für eine Gruppe der Formel (20).

Bevorzugte Ausführungsformen der Gruppe der Formel (2) sind daher die Verbindungen der folgenden Formeln (23) bis (29), 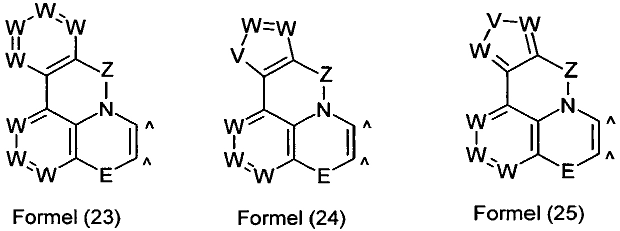 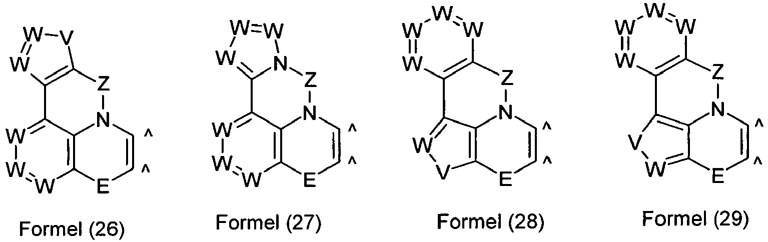 wobei ^ die Position der Verknüpfung in Formel (1) kennzeichnet und die weiteren verwendeten Symbole die oben genannten Bedeutungen aufweisen.

Wie oben bereits ausgeführt, können zwei benachbarte Gruppen W auch für eine Gruppe der oben genannten Formel (18) oder (19) stehen.

In einer weiteren bevorzugten Ausführungsform der Gruppen der Formeln (13) bis (17), (20) bis (22) und (23) bis (29) steht pro Cyclus insgesamt maximal ein Symbol W für N, und die verbleibenden Symbole W, die nicht für eine Gruppe der Formel (18) oder (19) stehen, stehen für CR. In einer besonders bevorzugten Ausführungsform der Erfindung stehen alle Symbole W, die nicht für eine Gruppe der Formel (18) oder (19) stehen, für CR. Besonders bevorzugte Gruppen der Formel (2) sind daher die Gruppen gemäß den folgenden Formeln (23a) bis (29a), 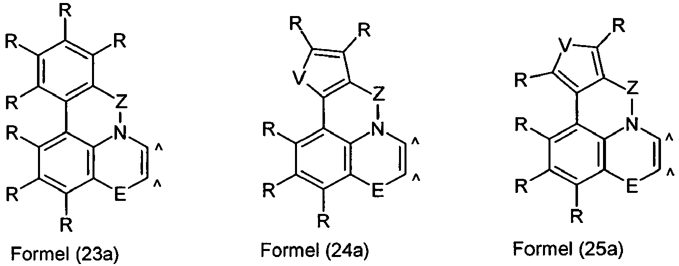 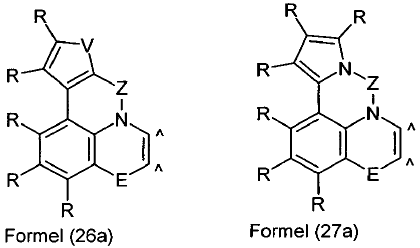 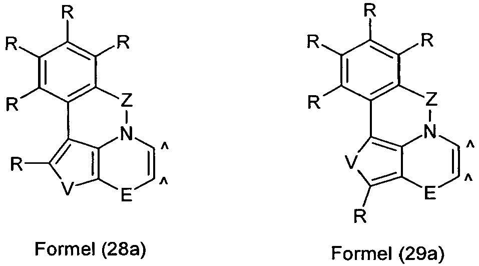 wobei die verwendeten Symbole die oben genannten Bedeutungen aufweisen.

Ganz besonders bevorzugt sind die Verbindungen gemäß den folgenden Formeln (23b) bis (29b), 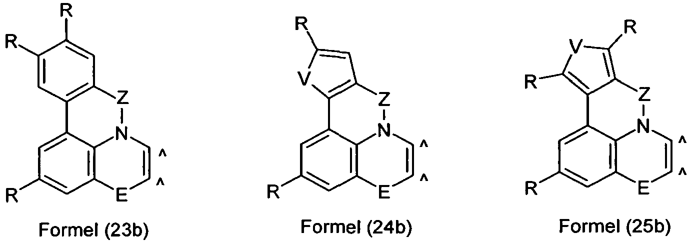 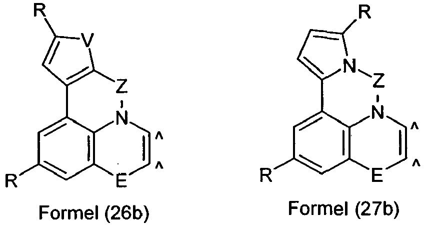 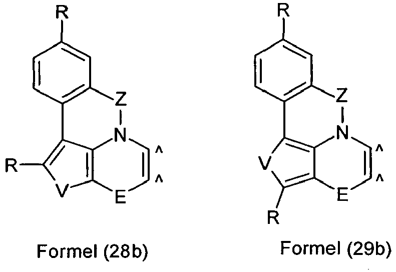 wobei die verwendeten Symbole die oben genannten Bedeutungen aufweisen.

Ganz besonders bevorzugte Gruppen der Formel (2) sind die Gruppen der Formel (23) bzw. (23a) bzw. (23b).

Dabei steht in den Formeln (23) bis (29), (23a) bis (29a) und (23b) bis (29b) Z bevorzugt für C=O.

Weiterhin steht in den Formeln (23) bis (29), (23a) bis (29a) und (23b) bis (29b) E bevorzugt für eine Einfachbindung.

Besonders bevorzugt steht in den Formeln (23) bis (29), (23a) bis (29a) und (23b) bis (29b) Z für C=O und gleichzeitig E für eine Einfachbindung.

Weiterhin ist es bevorzugt, falls zwei benachbarte Gruppen W für eine Gruppe der Formel (18) oder (19) stehen, dass dann maximal eine Gruppe G für N steht. Besonders bevorzugt stehen alle Gruppen G für CR. Weiterhin ist es bevorzugt, wenn zwei benachbarte Gruppen W für eine Gruppe der Formel (19) stehen, dass dann in der Gruppe der Formel (19) E für CR₂, C=O oder NR steht, insbesondere für CR₂ oder NR.

Die Verbindungen der oben genannten Formeln (5) bis (10) bzw. die bevorzugten Ausführungsformen können beliebig mit den Gruppen der oben genannten Formeln (23) bis (29) bzw. die bevorzugten Ausführungsformen kombiniert werden.

Geeignete Verbindungen sind daher die in folgenden Tabelle aufgeführten Verbindungen:

| Verbindung | Gruppe der Formel (2) |
|---|---|
| Formel (5) | Formel (23) |
| Formel (5) | Formel (24) |
| Formel (5) | Formel (25) |
| Formel (5) | Formel (26) |
| Formel (5) | Formel (27) |
| Formel (5) | Formel (28) |
| Formel (5) | Formel (29) |
| Formel (6) | Formel (23) |
| Formel (6) | Formel (24) |
| Formel (6) | Formel (25) |
| Formel (6) | Formel (26) |
| Formel (6) | Formel (27) |
| Formel (6) | Formel (28) |
| Formel (6) | Formel (29) |
| Formel (7) | Formel (23) |
| Formel (7) | Formel (24) |
| Formel (7) | Formel (25) |
| Formel (7) | Formel (26) |
| Formel (7) | Formel (27) |
| Formel (7) | Formel (28) |
| Formel (7) | Formel (29) |
| Formel (8) | Formel (23) |
| Formel (8) | Formel (24) |
| Formel (8) | Formel (25) |
| Formel (8) | Formel (26) |
| Formel (8) | Formel (27) |
| Formel (8) | Formel (28) |
| Formel (8) | Formel (29) |
| Formel (9) | Formel (23) |
| Formel (9) | Formel (24) |
| Formel (9) | Formel (25) |
| Formel (9) | Formel (26) |
| Formel (9) | Formel (27) |
| Formel (9) | Formel (28) |
| Formel (9) | Formel (29) |
| Formel (10) | Formel (23) |
| Formel (10) | Formel (24) |
| Formel (10) | Formel (25) |
| Formel (10) | Formel (26) |
| Formel (10) | Formel (27) |
| Formel (10) | Formel (28) |
| Formel (10) | Formel (29) |

Bevorzugte Verbindungen sind die in folgenden Tabelle aufgeführten Verbindungen:

| Verbindung | Gruppe der Formel (2) |
|---|---|
| Formel (5a) | Formel (23a) |
| Formel (5a) | Formel (24a) |
| Formel (5a) | Formel (25a) |
| Formel (5a) | Formel (26a) |
| Formel (5a) | Formel (27a) |
| Formel (5a) | Formel (28a) |
| Formel (5a) | Formel (29a) |
| Formel (6a) | Formel (23a) |
| Formel (6a) | Formel (24a) |
| Formel (6a) | Formel (25a) |
| Formel (6a) | Formel (26a) |
| Formel (6a) | Formel (27a) |
| Formel (6a) | Formel (28a) |
| Formel (6a) | Formel (29a) |
| Formel (7a) | Formel (23a) |
| Formel (7a) | Formel (24a) |
| Formel (7a) | Formel (25a) |
| Formel (7a) | Formel (26a) |
| Formel (7a) | Formel (27a) |
| Formel (7a) | Formel (28a) |
| Formel (7a) | Formel (29a) |
| Formel (8a) | Formel (23a) |
| Formel (8a) | Formel (24a) |
| Formel (8a) | Formel (25a) |
| Formel (8a) | Formel (26a) |
| Formel (8a) | Formel (27a) |
| Formel (8a) | Formel (28a) |
| Formel (8a) | Formel (29a) |
| Formel (9a) | Formel (23a) |
| Formel (9a) | Formel (24a) |
| Formel (9a) | Formel (25a) |
| Formel (9a) | Formel (26a) |
| Formel (9a) | Formel (27a) |
| Formel (9a) | Formel (28a) |
| Formel (9a) | Formel (29a) |
| Formel (10a) | Formel (23a) |
| Formel (10a) | Formel (24a) |
| Formel (10a) | Formel (25a) |
| Formel (10a) | Formel (26a) |
| Formel (10a) | Formel (27a) |
| Formel (10a) | Formel (28a) |
| Formel (10a) | Formel (29a) |

Besonders bevorzugte Verbindungen sind die in folgenden Tabelle aufgeführten Verbindungen:

| Verbindung | Gruppe der Formel (2) |
|---|---|
| Formel (5b) | Formel (23b) |
| Formel (5b) | Formel (24b) |
| Formel (5b) | Formel (25b) |
| Formel (5b) | Formel (26b) |
| Formel (5b) | Formel (27b) |
| Formel (5b) | Formel (28b) |
| Formel (5b) | Formel (29b) |
| Formel (6b) | Formel (23b) |
| Formel (6b) | Formel (24b) |
| Formel (6b) | Formel (25b) |
| Formel (6b) | Formel (26b) |
| Formel (6b) | Formel (27b) |
| Formel (6b) | Formel (28b) |
| Formel (6b) | Formel (29b) |
| Formel (7b) | Formel (23b) |
| Formel (7b) | Formel (24b) |
| Formel (7b) | Formel (25b) |
| Formel (7b) | Formel (26b) |
| Formel (7b) | Formel (27b) |
| Formel (7b) | Formel (28b) |
| Formel (7b) | Formel (29b) |
| Formel (8b) | Formel (23b) |
| Formel (8b) | Formel (24b) |
| Formel (8b) | Formel (25b) |
| Formel (8b) | Formel (26b) |
| Formel (8b) | Formel (27b) |
| Formel (8b) | Formel (28b) |
| Formel (8b) | Formel (29b) |
| Formel (9b) | Formel (23b) |
| Formel (9b) | Formel (24b) |
| Formel (9b) | Formel (25b) |
| Formel (9b) | Formel (26b) |
| Formel (9b) | Formel (27b) |
| Formel (9b) | Formel (28b) |
| Formel (9b) | Formel (29b) |
| Formel (10b) | Formel (23b) |
| Formel (10b) | Formel (24b) |
| Formel (10b) | Formel (25b) |
| Formel (10b) | Formel (26b) |
| Formel (10b) | Formel (27b) |
| Formel (10b) | Formel (28b) |
| Formel (10b) | Formel (29b) |

Unter diesen Verbindungen sind Verbindungen der Formel (5) bis (10) bevorzugt, welche als Gruppe der Formel (2) eine Gruppe der Formel (23) enthalten, besonders bevorzugt Verbindungen der Formel (5a) bis (10a), welche als Gruppe der Formel (2) eine Gruppe der Formel (23a) enthalten, und ganz besonders bevorzugt Verbindungen der Formel (5b) bis (10b), welche als Gruppe der Formel (2) eine Gruppe der Formel (23b) enthalten.

Ganz besonders bevorzugte Verbindungen sind somit die Verbindungen der folgenden Formeln (5c) bis (10c), 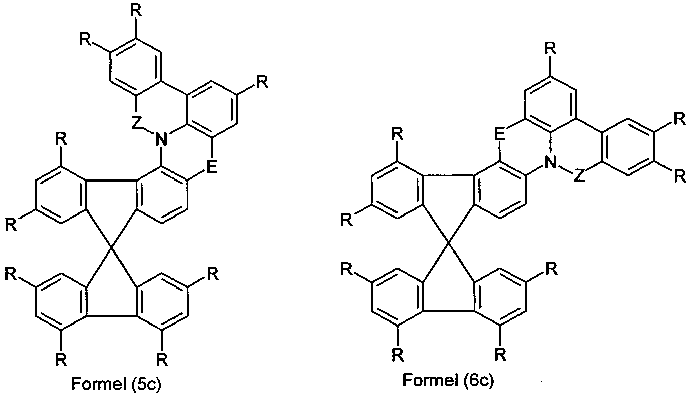 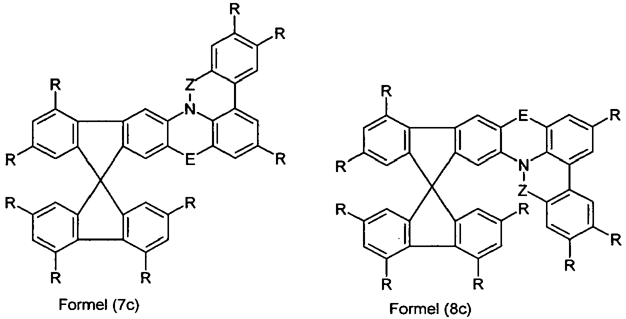 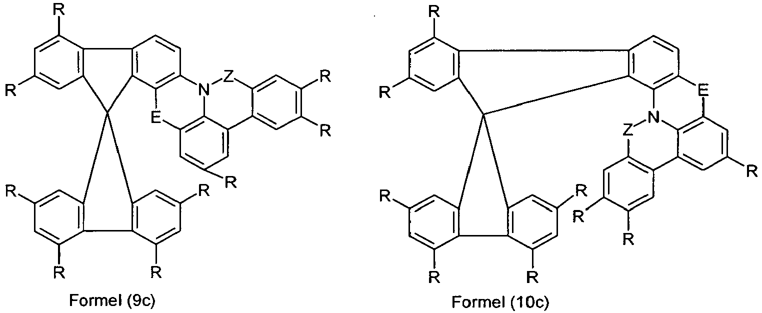 wobei die verwendeten Symbole die oben genannten Bedeutungen aufinreisen.

Ganz besonders bevorzugt sind die Verbindungen der folgenden Formeln (5d) bis (10d), 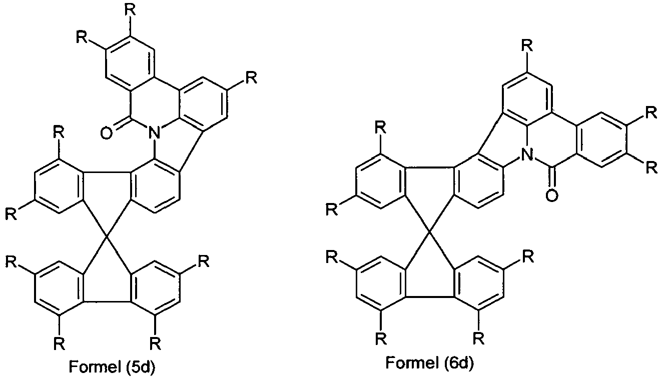 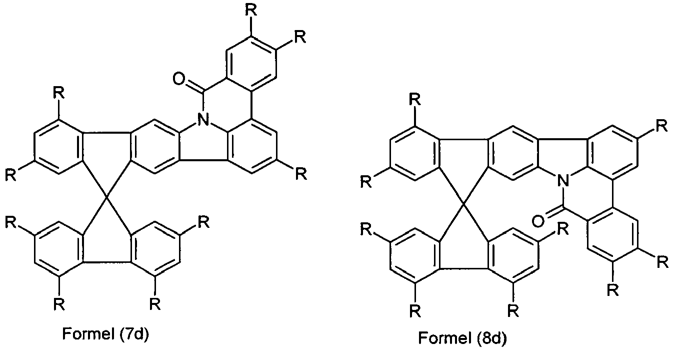 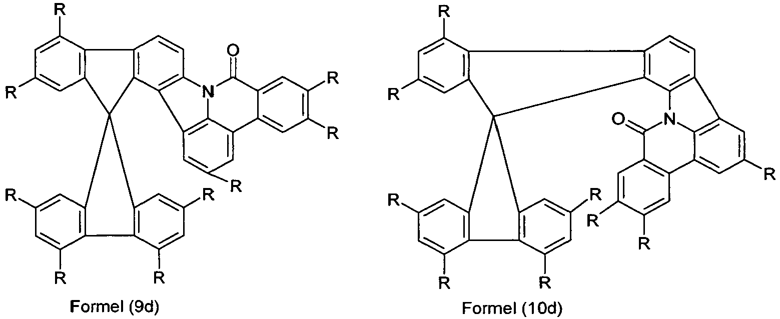 wobei die verwendeten Symbole die oben genannten Bedeutungen aufweisen.

In einer bevorzugten Ausführungsform der Erfindung ist R in den oben genannten Formeln gleich oder verschieden bei jedem Auftreten ausgewählt aus der Gruppe bestehend aus H, D, F, Cl, Br, CN, N(Ar³)₂, C(=O)Ar³, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenyl- oder Alkinylgruppe mit 2 bis 10 C-Atomen, die jeweils mit einem oder mehreren Resten R¹ substituiert sein kann, wobei eine oder mehrere nicht-benachbarte CH₂-Gruppen durch O ersetzt sein können und wobei ein oder mehrere H-Atome durch D oder F ersetzt sein können, oder einem aromatischen oder heteroaromatischen Ringsystem mit 5 bis 30 aromatischen Ringatomen, das jeweils mit einem oder mehreren Resten R¹ substituiert sein kann.

In einer besonders bevorzugten Ausführungsform der Erfindung ist R in den oben genannten Formeln gleich oder verschieden bei jedem Auftreten ausgewählt aus der Gruppe bestehend aus H, D, F, CN, N(Ar³)₂, einer geradkettigen Alkylgruppe mit 1 bis 4 C-Atomen oder einer verzweigten Alkylgruppe mit 3 oder 4 C-Atomen oder einer cyclischen Alkylgruppe mit 5 oder 6 C-Atomen, die jeweils mit einem oder mehreren Resten R¹ substituiert sein kann, wobei ein oder mehrere H-Atome durch D oder F ersetzt sein können, oder einem aromatischen oder heteroaromatischen Ringsystem mit 6 bis 24 aromatischen Ringatomen, das jeweils mit einem oder mehreren Resten R¹ substituiert sein kann.

In einer ganz besonders bevorzugten Ausführungsform der Erfindung ist R in den oben genannten Formeln gleich oder verschieden bei jedem Auftreten ausgewählt aus der Gruppe bestehend aus H oder einem aromatischen oder heteroaromatischen Ringsystem mit 6 bis 24, bevorzugt 6 bis 18 aromatischen Ringatomen, das jeweils mit einem oder mehreren Resten R¹ substituiert sein kann.

Wenn die Verbindungen der Formel (1) bzw. die bevorzugten Ausführungsformen als Elektronentransportmaterial verwendet werden, ist es bevorzugt, wenn die Gruppe Z für C=O steht und/oder wenn mindestens einer der Reste R für ein aromatisches Ringsystem oder ein elektronenarmes heteroaromatisches Ringsystem steht. Elektronenarme heteroaromatische Gruppen sind erfindungsgemäß Fünfringheteroaromaten mit mindestens zwei Heteroatomen oder Sechsringheteroaromaten mit mindestens einem Heteroatom, an die jeweils noch ein oder mehrere aromatische oder heteroaromatische Gruppen ankondensiert sein können.

Wenn die Verbindungen der Formel (1) bzw. die bevorzugten Ausführungsformen als Matrixmaterial für einen phosphoreszierenden Emitter verwendet werden, ist es bevorzugt, wenn die Gruppe Z für C=O steht und/oder wenn mindestens einer der Reste R für ein substituiertes oder unsubstituiertes Carbazol, Indenocarbazol oder Indolocarbazol steht, welches jeweils über ein Kohlenstoffatom oder ein Stickstoffatom gebunden sein kann. Weiterhin ist es in diesem Fall bevorzugt, wenn die erfindungsgemäßen Verbindungen keine Aryl- oder Heteroarylgruppen aufweisen, in denen zwei oder mehr Sechsringaryl- oder -heteroarylgruppen direkt aneinander ankondensiert sind. Besonders bevorzugt enthält in diesem Fall die erfindungsgemäße Verbindung überhaupt keine Aryl- oder Heteroarylgruppen mit direkt aneinander ankondensierten Sechsringen.

In einer bevorzugten Ausführungsform der Erfindung stehen entweder alle Reste R für H, oder es stehen genau ein oder zwei Reste R für ein aromatisches oder heteroaromatisches Ringsystem mit 6 bis 18 aromatischen Ringatomen, das jeweils mit einem oder mehreren Resten R¹ substituiert sein kann, und die anderen Reste R stehen für H.

Wenn ein oder mehrere Reste R für ein aromatisches oder heteroaromatisches Ringsystem stehen, dann sind bevorzugte Reste R gleich oder verschieden bei jedem Auftreten ausgewählt aus der Gruppe bestehend aus Phenyl, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1- oder 2-Naphthyl, Anthracen, Phenanthren, Triphenylen, Pyren, Benzanthracen, Pyrrol, Furan, Thiophen, Indol, Benzofuran, Benzothiophen, 1-, 2- oder 3-Carbazol, 1-, 2- oder 3-Dibenzofuran, 1-, 2- oder 3-Dibenzothiophen, Indenocarbazol, Indolocarbazol, Pyrazol, Imidazol, Benzimidazol, Pyridin, Pyrimidin, Pyrazin, Pyridazin, Triazin oder Chinolin oder Kombinationen aus zwei oder drei dieser Gruppen, welche jeweils mit einem oder mehreren Resten R¹ substituiert sein können.

Bevorzugte aromatische oder heteroaromatische Ringsysteme R sind ausgewählt aus den Gruppen der folgenden Formeln R-1 bis R-53, 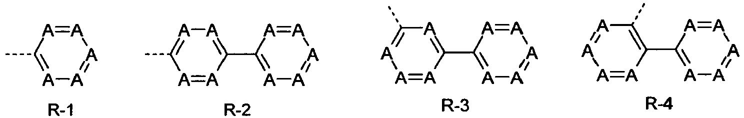 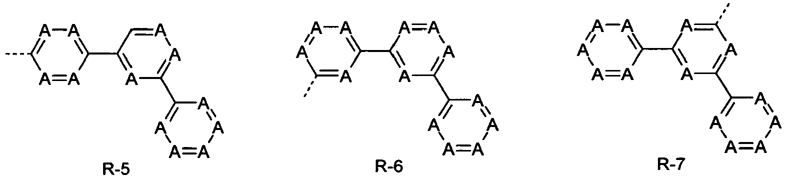 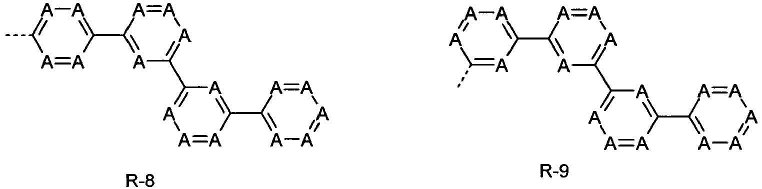 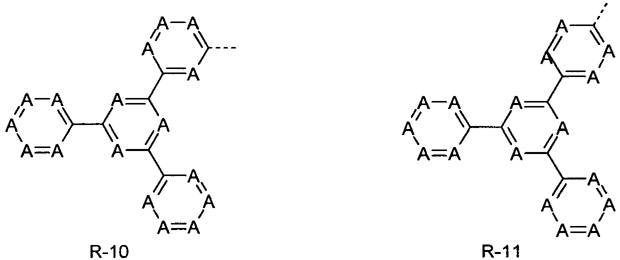 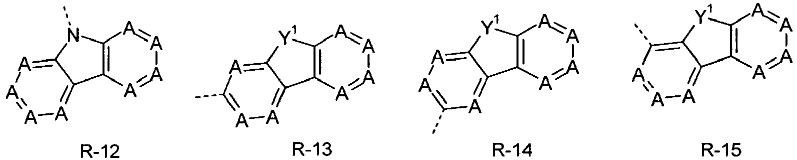 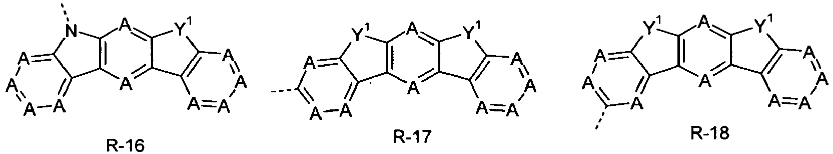 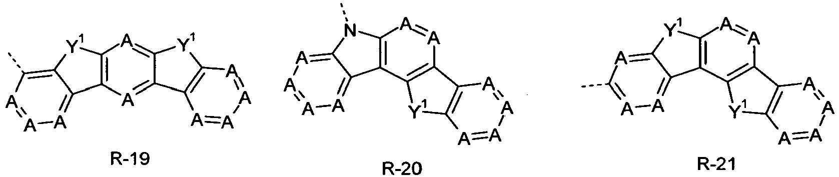 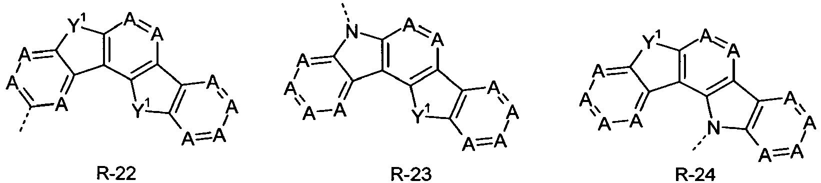 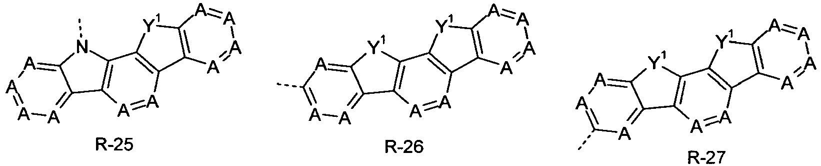 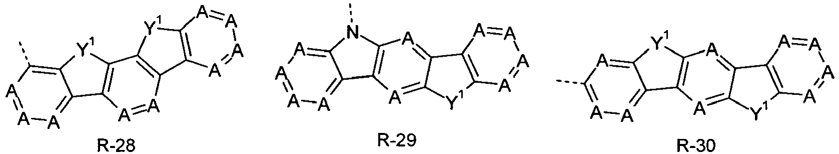 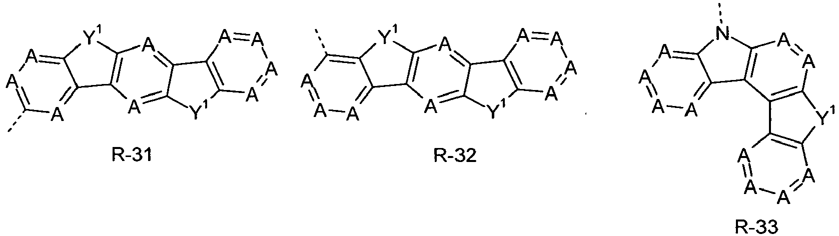 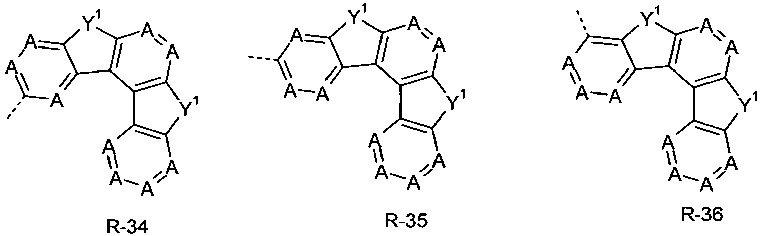 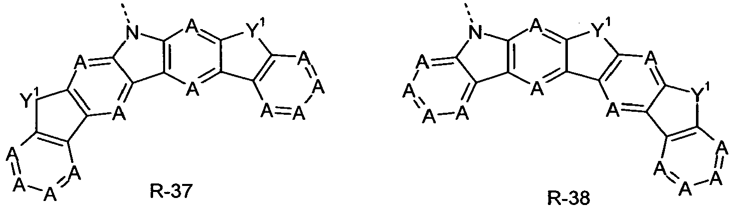 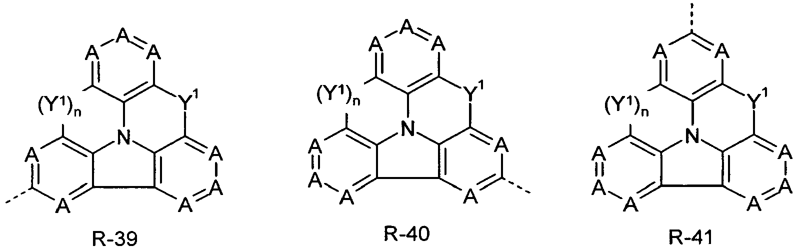 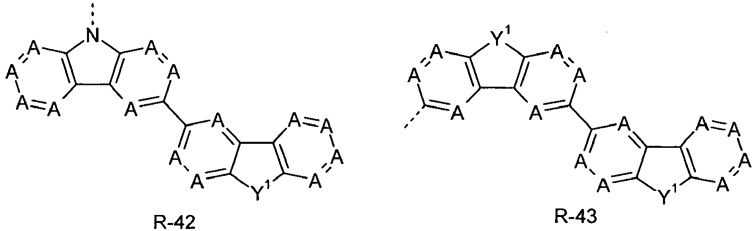 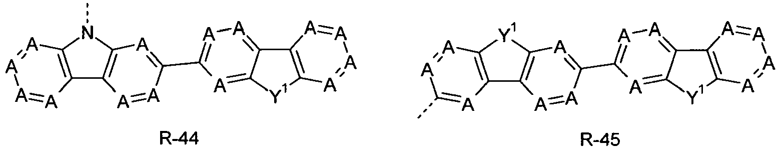 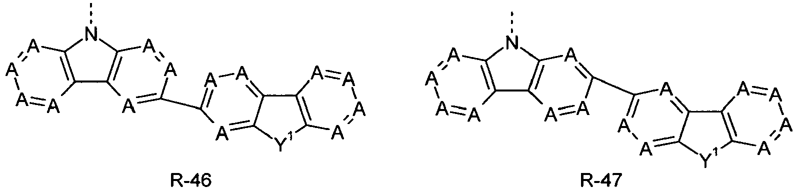 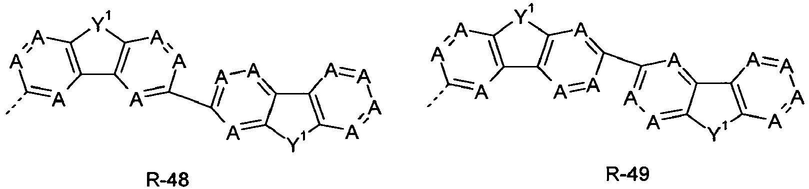 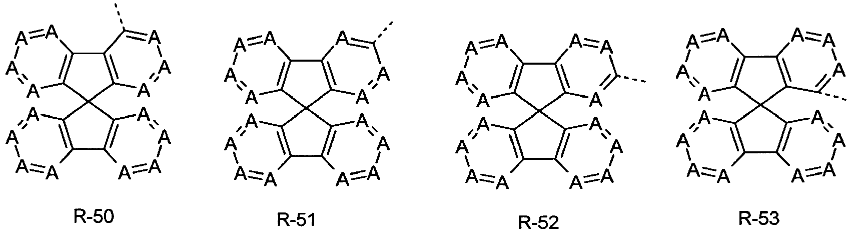 wobei R¹ die oben genannten Bedeutungen aufweist, die gestrichelte Bindung die Bindung an die Gruppe der Formel (1) darstellt und weiterhin gilt:
- A: ist bei jedem Auftreten gleich oder verschieden CR¹ oder N, wobei maximal 3 Symbole X pro Cyclus für N stehen;
- Y¹: ist bei jedem Auftreten gleich oder verschieden C(R¹)₂, NR¹, O oder S;
- n: ist 0 oder 1, wobei n gleich 0 bedeutet, dass an dieser Position keine Gruppe Y¹ gebunden ist und an den entsprechenden Kohlenstoffatomen statt dessen Reste R¹ gebunden sind.

Der oben genannte und auch im Folgenden verwendete Begriff "pro Cyclus" bezieht sich im Sinne der vorliegenden Anmeldung auf jeden einzelnen in der Verbindung enthaltenen Ring, also auf jeden einzelnen 5-bzw. 6-Ring.

In einer bevorzugten Ausführungsform der Gruppe R-1 stehen kein, ein, zwei oder drei Symbole A für N. Es handelt sich dabei besonders bevorzugt um Phenyl der Formel R-1a, Pyrimidin der Formel R-1b oder Triazin der Formel R-1c, 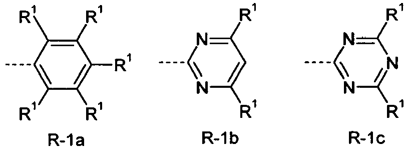 wobei R¹ die oben genannten Bedeutungen aufweist und in Formel R-1a insbesondere für H oder ein aromatisches oder heteroaromatisches Ringsystem mit 6 bis 18 aromatischen Ringatomen steht, welches mit einem oder mehreren Resten R² substituiert sein kann, und in Formel R-1b und R-1c insbesondere jeweils für ein aromatisches oder heteroaromatisches Ringsystem mit 6 bis 18 aromatischen Ringatomen steht, welches mit einem oder mehreren Resten R² substituiert sein kann.

In bevorzugten Gruppen der oben genannten Formeln R-2 bis R-53 steht maximal ein Symbol A pro Cyclus für N. Besonders bevorzugt steht das Symbol A gleich oder verschieden bei jedem Auftreten für CR¹, insbesondere für CH.

Wenn die oben genannten Gruppen für R-1 bis R-53 mehrere Gruppen Y¹ aufweisen, so kommen hierfür alle Kombinationen aus der Definition von Y¹ in Frage. Bevorzugt sind Gruppen, in denen eine Gruppe Y¹ für NR¹ und die andere Gruppe Y¹ für C(R¹)₂ steht oder in denen beide Gruppen Y¹ für NR¹ stehen oder in denen beide Gruppen Y¹ für O stehen.

In einer weiteren bevorzugten Ausführungsform der Erfindung steht mindestens eine Gruppe Y¹ für C(R¹)₂ oder für NR¹.

Wenn Y¹ für NR¹ steht, steht der Substituent R¹, der an das Stickstoffatom gebunden ist, bevorzugt für ein aromatisches oder heteroaromatisches Ringsystem mit 5 bis 24 aromatischen Ringatomen, welches auch durch einen oder mehrere Reste R² substituiert sein kann. In einer besonders bevorzugten Ausführungsform steht dieser Substituent R¹ gleich oder verschieden bei jedem Auftreten für ein aromatisches oder heteroaromatisches Ringsystem mit 6 bis 24 aromatischen Ringatomen, welches keine kondensierten Arylgruppen aufweist und welches keine kondensierten Heteroarylgruppen, in welchen zwei oder mehr aromatische bzw. heteroaromatische 6-Ring-Gruppen direkt aneinander ankondensiert sind, aufweist und welches jeweils auch durch einen oder mehrere Reste R² substituiert sein kann. Besonders bevorzugt sind Phenyl, Biphenyl, Terphenyl und Quaterphenyl.

Wenn Y¹ für C(R¹)₂ steht, steht R¹ bevorzugt gleich oder verschieden bei jedem Auftreten für eine lineare Alkylgruppe mit 1 bis 10 C-Atomen, bevorzugt 1 bis 4 C-Atomen, oder für eine verzweigte Alkylgruppe mit 3 bis 10 C-Atomen, bevorzugt 3 oder 4 C-Atomen oder für ein aromatisches oder heteroaromatisches Ringsystem mit 5 bis 24 aromatischen Ringatomen, bevorzugt 6 bis 18 aromatischen Ringatomen, welches auch durch einen oder mehrere Reste R² substituiert sein kann. Ganz besonders bevorzugt steht R¹ für eine Methylgruppe oder für eine Phenylgruppe. Dabei können die Reste R¹ auch miteinander ein Ringsystem bilden, was zu einem Spirosystem führt.

In einer weiteren bevorzugten Ausführungsform der Erfindung steht R für eine Triarylamingruppe, welche durch einen oder mehrere Reste R¹ substituiert sein kann. Diese ist bevorzugt ausgewählt aus den Strukturen der folgenden Formel R-54, 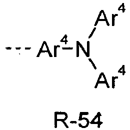 wobei die gestrichelte Bindung die Verknüpfung mit dem Grundgerüst andeutet und Ar⁴ gleich oder verschieden bei jedem Auftreten für ein aromatisches oder heteroaromatisches Ringsystem mit jeweils 5 bis 18 aromatischen Ringatomen, bevorzugt mit jeweils 6 bis 12 aromatischen Ringatomen steht, welches jeweils mit einem oder mehreren Resten R¹ substituiert sein kann.

Dabei haben in Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Die oben genannten bevorzugten Ausführungsformen können beliebig miteinander kombiniert werden. In einer besonders bevorzugten Ausführungsform der Erfindung treten die oben genannten Bevorzugungen gleichzeitig auf.

Beispiele für bevorzugte Verbindungen gemäß den oben aufgeführten Ausführungsformen sind die Verbindungen der folgenden Strukturen.

| | |
|---|---|
| 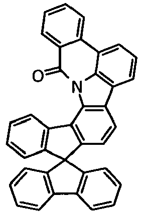 | 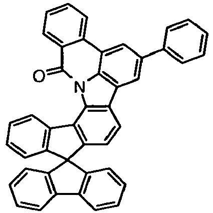 |
| (1) | (2) |
| 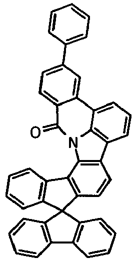 | 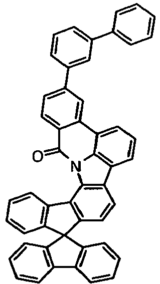 |
| (3) | (4) |
| 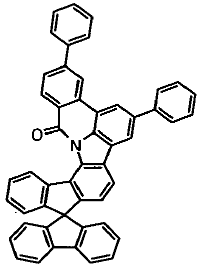 | 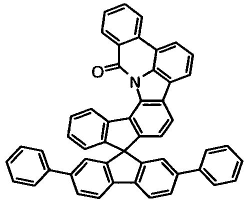 |
| (5) | (6) |
| 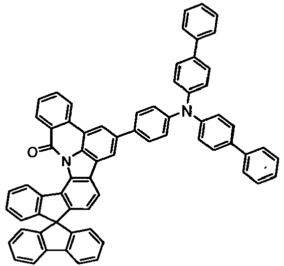 | 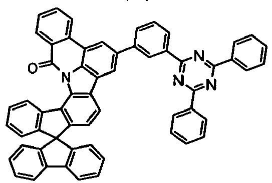 |
| (7) | (8) |
| 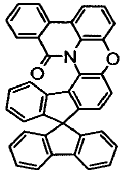 | 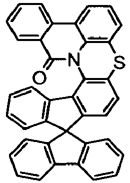 |
| (9) | (10) |
| 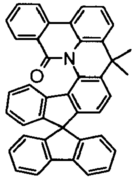 | 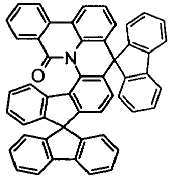 |
| (11) | (12) |
| 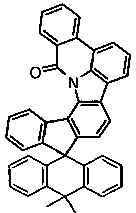 | 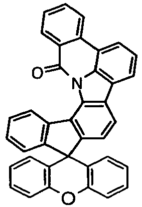 |
| (13) | (14) |
| 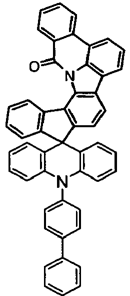 | 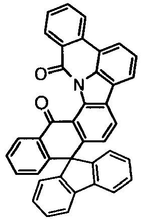 |
| (15) | (16) |
| 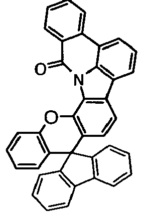 | 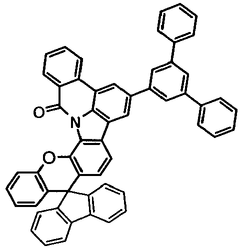 |
| (17) | (18) |
| 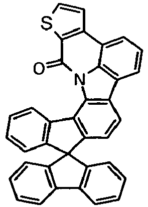 | 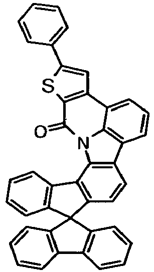 |
| (19) | (20) |
| 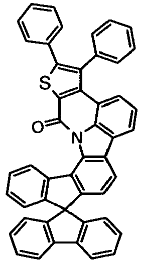 | 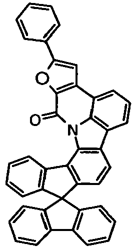 |
| (21) | (22) |
| 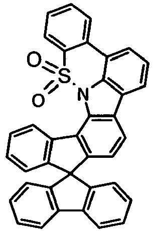 | 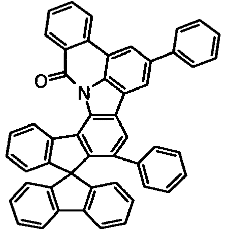 |
| (23) | (24) |
| 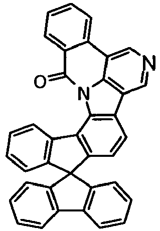 | 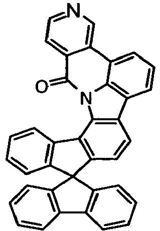 |
| (25) | (26) |
| 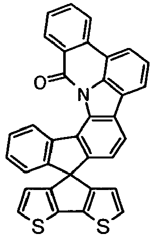 | 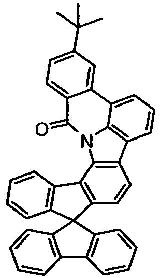 |
| (27) | (28) |
| 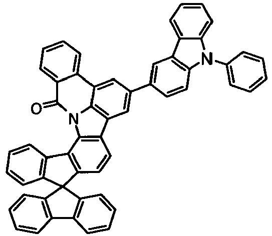 | 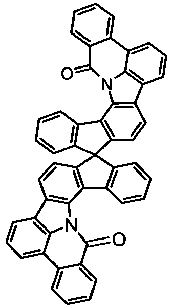 |
| (29) | (30) |
| 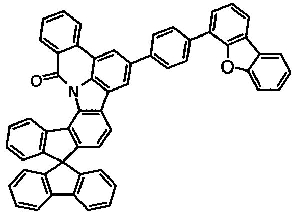 | 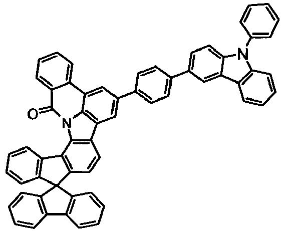 |
| (31) | (32) |
| 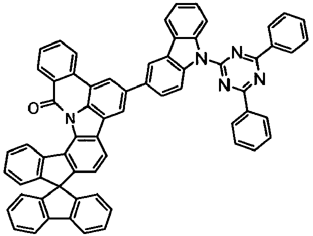 | 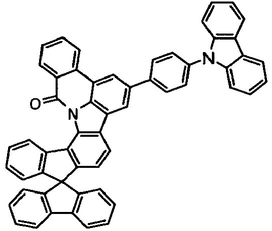 |
| (33) | (34) |
| 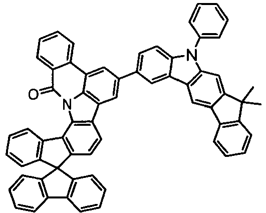 | 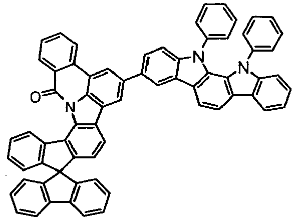 |
| (35) | (36) |
| 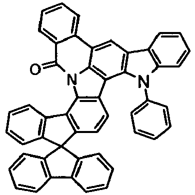 | 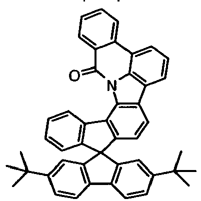 |
| (37) | (38) |
| 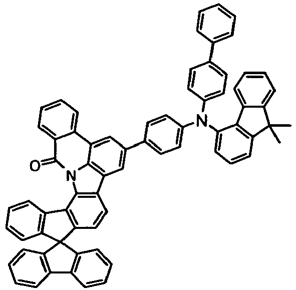 | 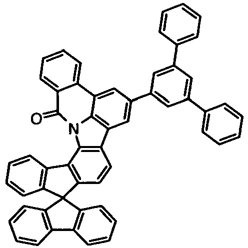 |
| (39) | (40) |
| 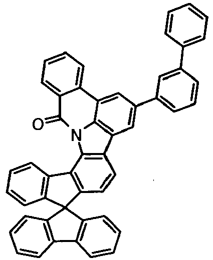 | 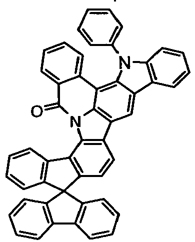 |
| (41) | (42) |
| 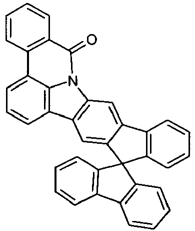 | 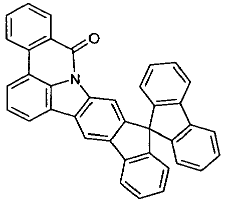 |
| (43) | (44) |
| 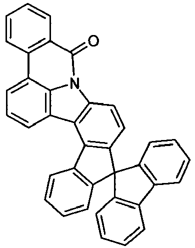 | 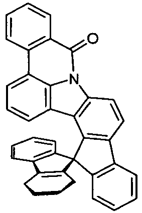 |
| (45) | (46) |
| 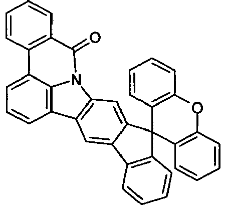 | 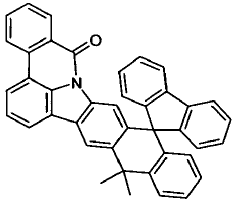 |
| (47) | (48) |
| 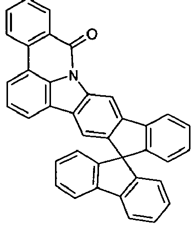 | 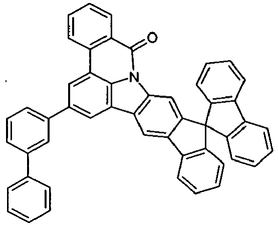 |
| (49) | (50) |
| 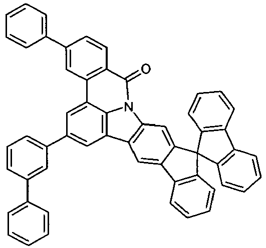 | 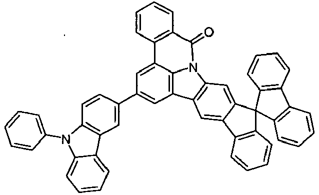 |
| (51) | (52) |
| 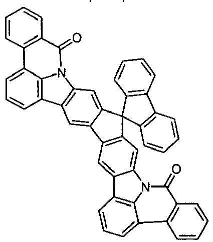 | 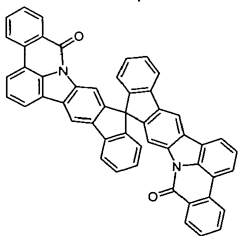 |
| (53) | (54) |
| 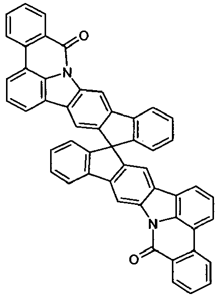 | 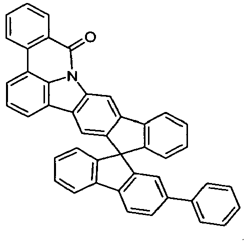 |
| (55) | (56) |
| 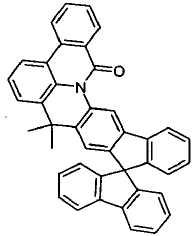 | 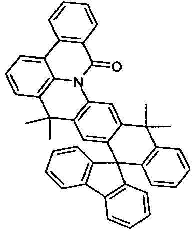 |
| (57) | (58) |
| 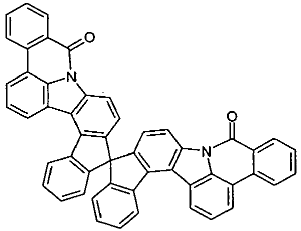 | 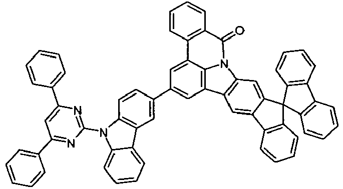 |
| (59) | (60) |
| 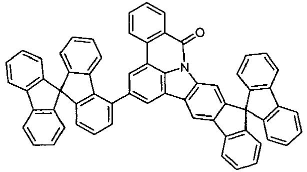 | 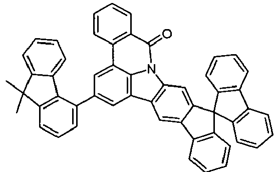 |
| (61) | (62) |
| 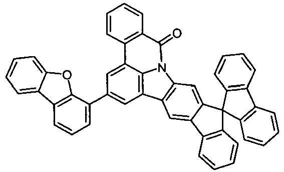 | 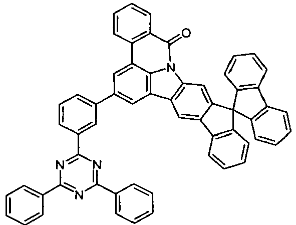 |
| (63) | (64) |
| 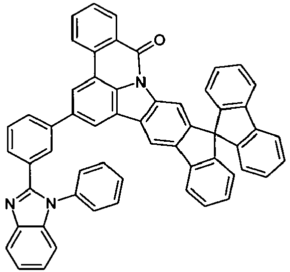 | 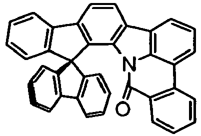 |
| (65) | (66) |
| 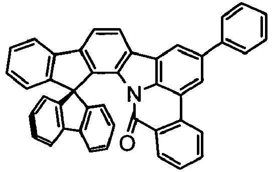 | 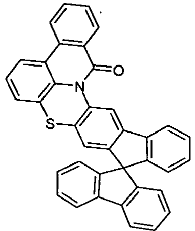 |
| (67) | (68) |
| 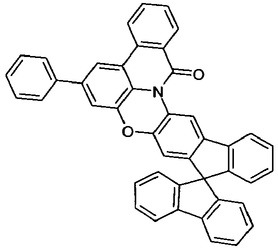 | 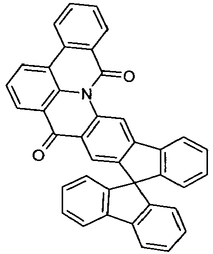 |
| (69) | (70) |
| 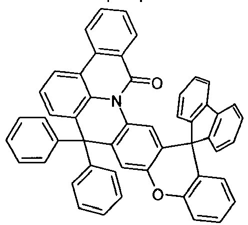 | 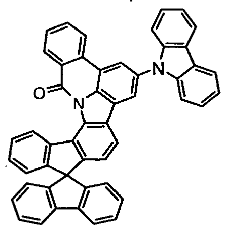 |
| (71) | (72) |
| 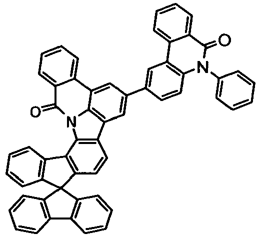 | 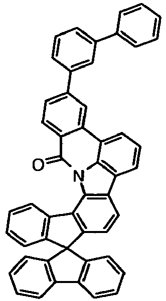 |
| (73) | (74) |
| 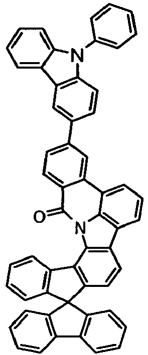 | 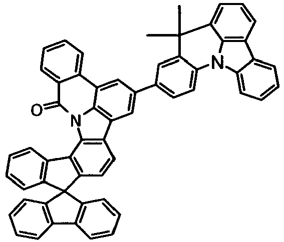 |
| (75) | (76) |
| 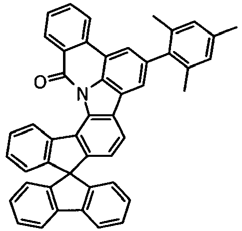 | 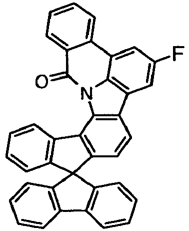 |
| (77) | (78) |
| 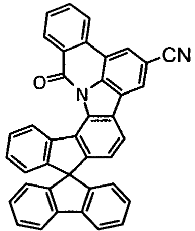 | 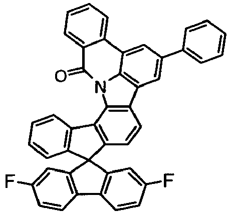 |
| (79) | (80) |
| 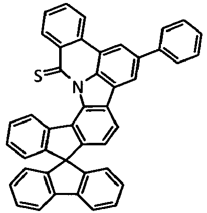 | 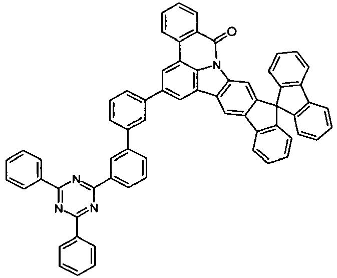 |
| (81) | (82) |
| 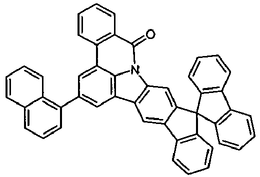 | 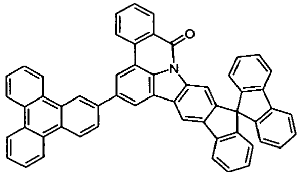 |
| (83) | (84) |
| 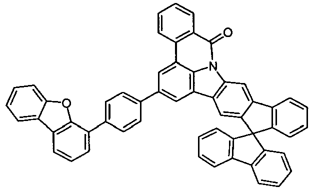 | 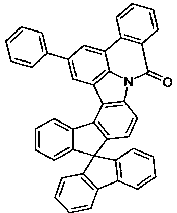 |
| (85) | (86) |
| 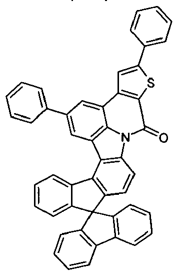 | 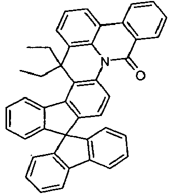 |
| (87) | (88) |
| 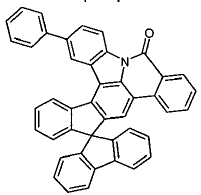 | 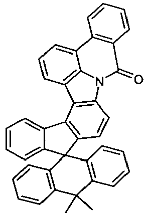 |
| (89) | (90) |
| 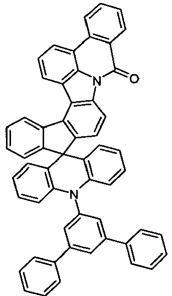 | 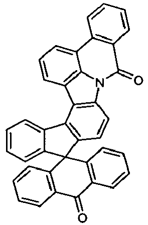 |
| (91) | (92) |
| 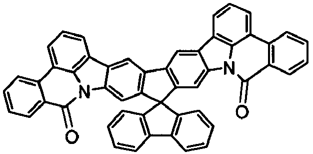 | 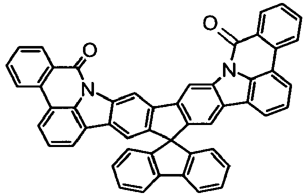 |
| (93) | (94) |

Die Verbindungen der Formel (1) bzw. die bevorzugten Ausführungsformen können nach dem Fachmann bekannten Syntheseschritten dargestellt werden, wie in Schema 1 schematisch dargestellt.

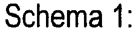

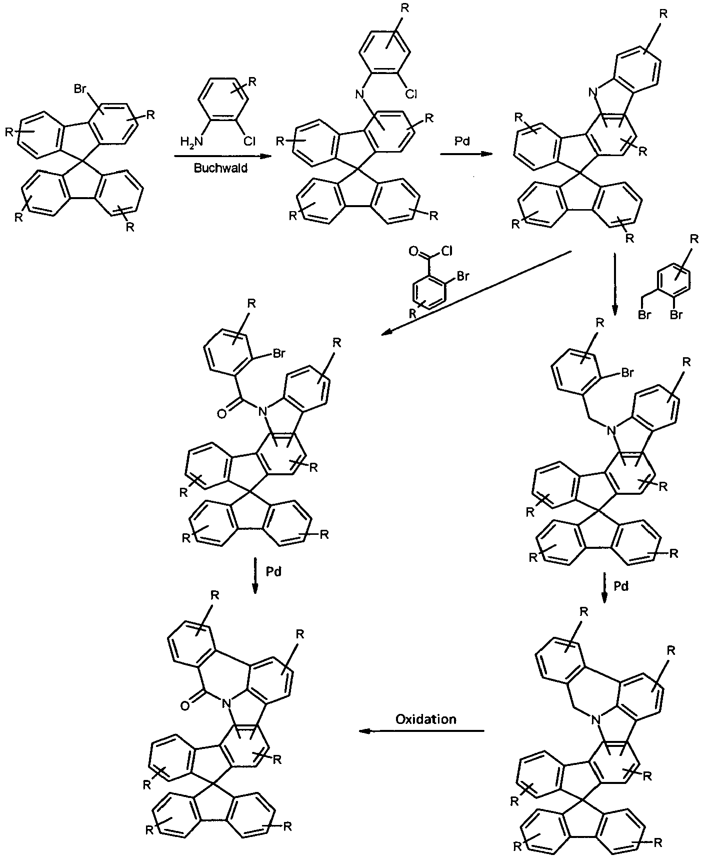

Die Synthese geht aus von einem Halogen-funktionalisierten, insbesondere Brom-funktionalisierten Spirobifluorenderivat. Wenn die Verbindungen Gruppen A¹ oder A² enthalten, die keine Einfachbindungen sind, so geht man von den entsprechenden funktionalisierten Spiroverbindungen mit Gruppen A¹ und A² aus. Dieses wird in einer C-N-Kupplungsreaktion, beispielsweise einer Hartwig-Buchwald-Kupplung, mit einem ortho-Halogenamino-substituierten Aromaten oder Heteroaromaten, beispielsweise einem ortho-Chloraminobenzol-Derivat umgesetzt, gefolgt von einer Palladium-katalysierten Ringschlussreaktion zum entsprechenden Spirocarbazolderivat. Umsetzung mit einem ortho-Halogen-carbonsäurechlorid zum entsprechenden Amid, gefolgt von einer Palladium-katalysierten Ringschlussreaktion führt zu den erfindungsgemäßen Verbindungen.

Alternativ kann die Umsetzung auch mit einem ortho-Halogen-benzylbromid bzw. einer entsprechenden heteroaromatischen Verbindung gefolgt von einer Palladium-katalysierten Ringschlussreaktion und Oxidation des cyclischen Amins zum Lactam erfolgen. Substituierte Verbindungen sind durch Verwendung entsprechend substituierter Edukte zugänglich.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind, können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten erfindungsgemäßen Verbindungen, wobei eine oder mehrere Bindungen der erfindungsgemäßen Verbindung zum Polymer, Oligomer oder Dendrimer vorhanden sind. Je nach Verknüpfung der erfindungsgemäßen Verbindung bildet diese daher eine Seitenkette des Oligomers oder Polymers oder ist in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Homopolymere oder Copolymere, wobei die Einheiten gemäß Formel (1) bzw. die oben ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/22026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten. Außerdem können die Polymere entweder einpolymerisiert oder als Blend eingemischt Triplett-Emitter enthalten. Gerade die Kombination von Einheiten gemäß Formel (1') bzw. der oben ausgeführten bevorzugten Ausführungsformen mit Triplett-Emittern führt zu besonders guten Ergebnissen.

Weiterhin können die Verbindungen gemäß Formel (1) bzw. die oben ausgeführten bevorzugten Ausführungsformen auch weiter funktionalisiert werden und so zu erweiterten Strukturen umgesetzt werden. Hier ist als Beispiel die Umsetzung mit Arylboronsäuren gemäß Suzuki oder mit primären oder sekundären Aminen gemäß Hartwig-Buchwald zu nennen. So können die Verbindungen gemäß Formel (1) bzw. die oben ausgeführten bevorzugten Ausführungsformen auch direkt an phosphoreszierende Metallkomplexe oder auch an andere Metallkomplexe gebunden werden.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung und/oder ein weiteres Matrixmaterial. Geeignete emittierende Verbindungen und weitere Matrixmaterialien sind hinten im Zusammenhang mit der organischen Elektrolumineszenzvorrichtung aufgeführt. Diese weitere Verbindung kann auch polymer sein.

Die erfindungsgemäßen Verbindungen eignen sich für die Verwendung in einer elektronischen Vorrichtung, insbesondere in einer organischen Elektrolumineszenzvorrichtung.

Ein weiterer Gegenstand der vorliegenden Erfindung ist die Verwendung der oben ausgeführten erfindungsgemäßen Verbindungen gemäß Formel (1) bzw. der bevorzugten Ausführungsformen in einer elektronischen Vorrichtung, insbesondere in einer organischen Elektrolumineszenzvorrichtung.

Eine elektronische Vorrichtung im Sinne der vorliegenden Erfindung ist eine Vorrichtung, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen farbstoffsensibilisierten Solarzellen (O-DSSC), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices" (D. M. Koller et al., Nature Photonics 2008, 1-4), bevorzugt aber organischen Elektrolumineszenzvorrichtungen (OLEDs), besonders bevorzugt phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013).

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. die oben ausgeführten bevorzugten Ausführungsformen als Matrixmaterial für fluoreszierende oder phosphoreszierende Emitter, insbesondere für phosphoreszierende Emitter, und/oder in einer Lochblockierschicht und/oder in einer Elektronentransportschicht und/oder in einer elektronenblockierenden bzw. exzitonenblockierenden Schicht und/oder in einer Lochtransportschicht, je nach genauer Substitution.

In einer bevorzugten Ausführungsform der Erfindung wird die Verbindung gemäß Formel (1) bzw. die oben ausgeführten bevorzugten Ausführungsformen als Matrixmaterial für eine fluoreszierende oder phosphoreszierende Verbindung, insbesondere für eine phosphoreszierende Verbindung, in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Wenn die Verbindung gemäß Formel (1) bzw. die oben ausgeführten bevorzugten Ausführungsformen als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der Verbindung gemäß Formel (1) bzw. der oben ausgeführten bevorzugten Ausführungsformen und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der Verbindung gemäß Formel (1) bzw. der oben ausgeführten bevorzugten Ausführungsformen bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der Verbindung gemäß Formel (1) bzw. der oben ausgeführten bevorzugten Ausführungsformen als Matrixmaterial für einen phosphoreszierenden Emitter in Kombination mit einem weiteren Matrixmaterial. Besonders geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, oder Triphenylenderivate, z. B. gemäß WO 2012/048781. Ebenso kann ein weiterer phosphoreszierender Emitter, welcher kürzerwellig als der eigentliche Emitter emittiert, als Co-Host in der Mischung vorhanden sein oder eine Verbindung, die nicht oder nicht in wesentlichem Umfang am Ladungstransport teilnimmt, wie beispielsweise in WO 2010/108579 beschrieben.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 2005/033244, WO 2005/019373, US 2005/0258742, WO 2010/086089, WO 2011/157339, WO 2012/007086, WO 2012/163471, WO 2013/000531, WO 2013/020631, WO 2014/008982 und WO 2014/023377 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In einer weiteren bevorzugten Ausführungsform der Erfindung wird die Verbindung gemäß Formel (1) bzw. die oben ausgeführten bevorzugten Ausführungsformen als Elektronentransportmaterial in einer Elektronentransport- oder Elektroneninjektionsschicht eingesetzt. Dabei kann die emittierende Schicht fluoreszierend oder phosphoreszierend sein. Wenn die Verbindung als Elektronentransportmaterial eingesetzt wird, kann es bevorzugt sein, wenn sie dotiert ist, beispielsweise mit Alkalimetallkomplexen, wie z. B. LiQ (Lithiumhydroxychinolinat).

In nochmals einer weiteren bevorzugten Ausführungsform der Erfindung wird die Verbindung gemäß Formel (1) bzw. die oben ausgeführten bevorzugten Ausführungsformen in einer Lochblockierschicht eingesetzt. Unter einer Lochblockierschicht wird eine Schicht verstanden, die auf Kathodenseite direkt an eine emittierende Schicht angrenzt.

Es ist weiterhin möglich, die Verbindung gemäß Formel (1) bzw. die oben ausgeführten bevorzugten Ausführungsformen sowohl in einer Lochblockierschicht bzw. Elektronentransportschicht als auch als Matrix in einer emittierenden Schicht zu verwenden.

In nochmals einer weiteren Ausführungsform der Erfindung wird die Verbindung gemäß Formel (1) bzw. die oben ausgeführten bevorzugten Ausführungsformen in einer Lochtransportschicht bzw. in einer Elektronenblockierschicht bzw. Exzitonenblockierschicht eingesetzt.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden. Diese Verfahren eignen sich insbesondere auch für Oligomere, Dendrimere und Polymere.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich durch folgende überraschende Vorteile gegenüber dem Stand der Technik aus:
1. Die erfindungsgemäßen Verbindungen bzw. Verbindungen gemäß Formel (1) bzw. die oben ausgeführten bevorzugten Ausführungsformen, eingesetzt insbesondere als Matrixmaterial für phosphoreszierende Emitter, führen zu hohen Effizienzen sowie zu langen Lebensdauern. Dies gilt insbesondere, wenn die Verbindungen als Matrixmaterial für einen rot, gelb oder grün phosphoreszierenden Emitter eingesetzt werden.
2. Organische Elektrolumineszenzvorrichtungen enthaltend die erfindungsgemäßen Verbindungen weisen eine geringe Betriebsspannung auf. Dies führt zu hohen Leistungseffizienzen.
3. Die erfindungsgemäßen Verbindungen weisen eine hohe thermische Stabilität sowie hohe Glasübergangstemperaturen auf.
4. Auch bei Verwendung als Elektronentransportmaterial führen die erfindungsgemäßen Verbindungen zu sehr guten Eigenschaften in Bezug auf die Effizienz, die Lebensdauer und die Betriebsspannung von organischen Elektrolumineszenzvorrichtungen.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre durchgeführt. Die Edukte können von den Firmen ALDRICH bzw. ABCR bezogen werden. Die in eckigen Klammern zu den literaturbekannten Verbindungen angegebenen Nummern kennzeichnen die CAS-Nummern dieser Verbindungen.

### Synthesebeispiele

### Beispiel A: Synthese 1-Brom-spiro-9,9'-bifluoren, 3-Brom-spiro-9,9'-bifluoren, 4-Brom-spiro-9,9'-bifluoren, 4,4'-Dibrom-spiro-9,9'-bifluoren, 3,6-Dibrom-spiro-9,9'-bifluoren

### a) 1-Brom-spiro-9,9'-bifluoren

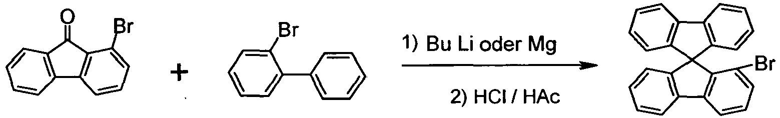

Aus mit Iod aktivierten 2.7 g (110 mmol) Magnesiumspänen und einer Mischung aus 25.6 g (110 mmol) 2-Brombiphenyl, 0.8 ml 1,2-Dichlorethan, 50 ml 1,2-Dimethoxyethan, 400 ml THF und 200 ml Toluol wird unter Begleitheizen mit einem 70 °C warmen Ölbad das entsprechende Grignard-Reagenz dargestellt. Nachdem das Magnesium vollständig abreagiert hat, lässt man auf Raumtemperatur erkalten und tropft dann eine Lösung von 25.9 g (100 mmol) 1-Brom-fluorenon [36804-63-4] in 500 ml THF zu, erwärmt die Reaktionsmischung für 4 h auf 50 °C und rührt dann 12 h bei Raumtemperatur nach. Man gibt 100 ml Wasser zu, rührt kurz nach, trennt die organische Phase ab und entfernt das Lösungsmittel im Vakuum. Der Rückstand wird in der Wärme bei 40 °C in 500 ml Eisessig suspendiert, die Suspension wird mit 0.5 ml konz. Schwefelsäure versetzt und anschließend 2 h bei 100 °C nachgerührt. Nach Erkalten saugt man vom ausgefallenen Feststoff ab, wäscht diesen einmal mit 100 ml Eisessig, dreimal mit je 100 ml Ethanol und kristallisiert abschließend aus Dioxan um. Ausbeute: 26.9 g (68 mmol), 68 %; Reinheit ca. 98 % n. ¹H-NMR.

| Br-Biphenyl | Br-Fluorenon | Produkt: Br-Spiro | Ausbeute |
|---|---|---|---|
| 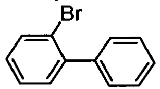 | 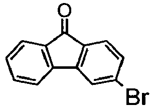 | 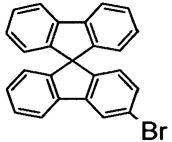 | 85 % |
| 2052-07-5 | 2041-19-2 | | |
| 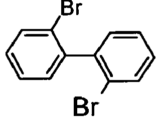 | 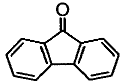 | 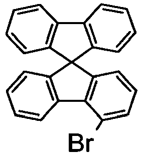 | 90% |
| 13029-09-9 | 486-25-9 | 1161009-88-6 | |
| 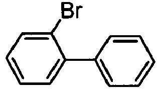 | 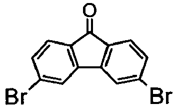 | 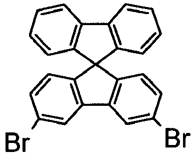 | 85% |
| 2052-07-5 | 216312-73-1 | | |
| 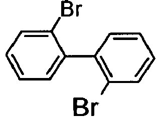 | 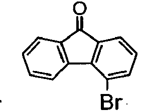 | 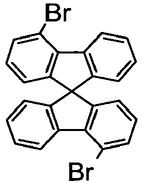 | 90% |
| 13029-09-9 | 4269-17-4 | 1257321-41-7 | |

### Beispiel 1a: (2-Chlorphenyl)-4-spiro-9,9'-bifluorenyl-amin

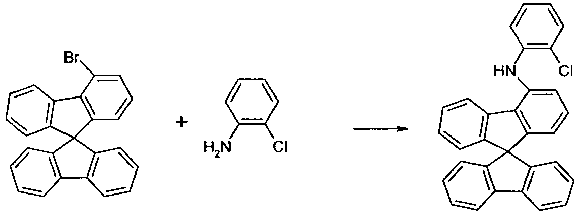

54 g (137 mmol) 4-Bromspiro-9,9'-bifluoren, 17.9 g (140 mmol) 2-Chloranilin, 68.2 g (710 mmol) Natrium-tert-butylat, 613 mg (3 mmol) Palladium-(II)acetat und 3.03 g (5 mmol) dppf werden in 1.3 L Toluol gelöst und 5 h unter Rückfluss gerührt. Das Reaktionsgemisch wird auf Raumtemperatur abgekühlt, mit Toluol erweitert und über Celite filtriert. Das Filtrat wird im Vakuum eingeengt und der Rückstand aus Toluol/Heptan kristallisiert. Das Produkt wird als farbloser Feststoff isoliert. Ausbeute: 52.2 g (118 mmol) 86% d.Th.

Analog können folgende Verbindungen hergestellt werden:

| | Edukt 1 | Edukt 2 | Produkt | Ausbeute |
|---|---|---|---|---|
| 1b | 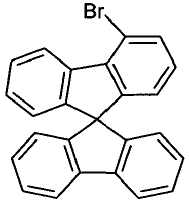 | 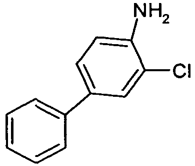 | 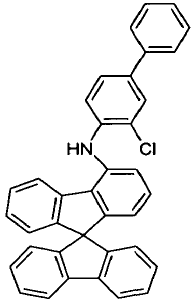 | 83% |
| | | 7285-66-7 | | |
| 1c | 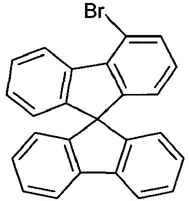 | 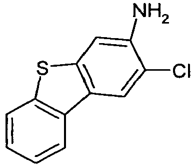 | 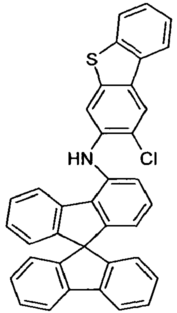 | 78% |
| | | 858426-71-8 | | |
| 1d | 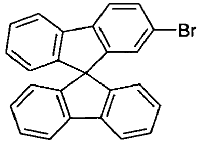 | 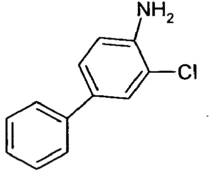 | 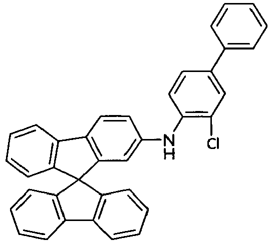 | 72% |
| | 171408-76-7 | 7285-66-7 | | |
| 1e | 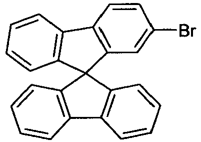 | 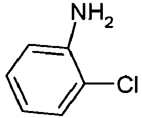 | 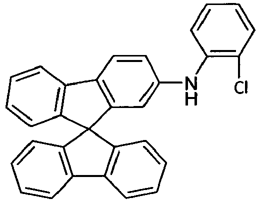 | 74% |
| | 171408-76-7 | 95-51-2 | | |
| 1f | 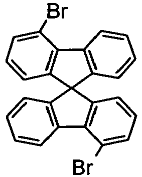 | 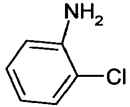 | 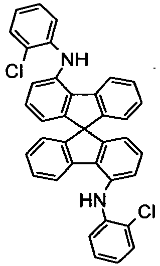 | 68% |
| | | 95-51-2 | | |
| 1g | 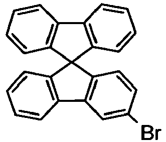 | 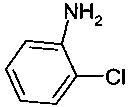 | 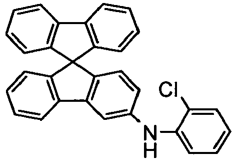 | 79% |
| | | 95-51-2 | | |
| 1h | 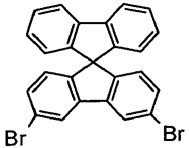 | 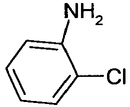 | 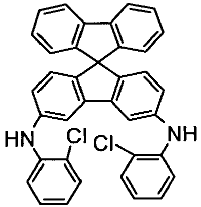 | 63% |
| | | 95-51-2 | | |
| 1i | 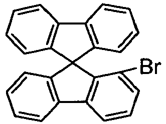 | 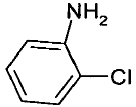 | 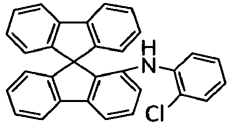 | 61% |
| | | 95-51-2 | | |

### Beispiel 2a: Spiro[9H-fluoren-9,7'(1'H)-indeno[1,2-a]carbazol]

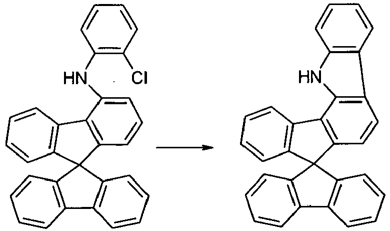

45 g (102 mmol) (2-Chlorphenyl)-4-spiro-9,9'-bifluorenyl-amin, 56 g (409 mmol) Kaliumcarbonat, 4.5 g (12 mmol) Tricyclohexylphosphintetrafluoroborat und 1.38 g (6 mmol) Palladium(II)acetat werden in 500 mL Dimethylacetamid suspendiert und 6 h unter Rückfluss gerührt. Nach Erkalten wird die Reaktionmischung mit 300 ml Wasser und 600 mL Dichlormethan. Man rührt 30 min. nach, trennt die organische Phase ab, filtriert diese über ein kurzes Celite-Bett und entfernt dann das Lösungsmittel im Vakuum. Das Rohprodukt wird mit Toluol heiß extrahiert und aus Toluol umkristallisiert. Das Produkt wird als beigefarbener Feststoff isoliert. Ausbeute: 32.5 g (80 mmol entsprechen 78 % d.Th.)

Analog können folgende Verbindungen hergestellt werden:

| | Edukt | Produkt | Ausbeute |
|---|---|---|---|
| 2b | 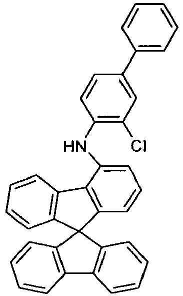 | 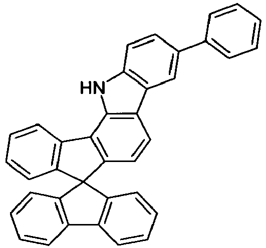 | 72% |
| 2c | 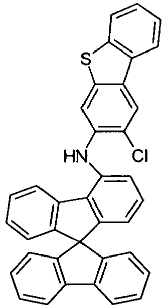 | 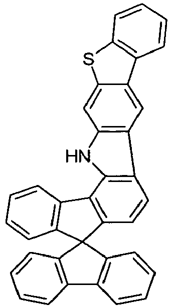 | 73% |
| 2d | 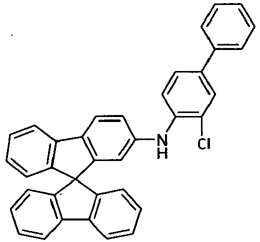 | 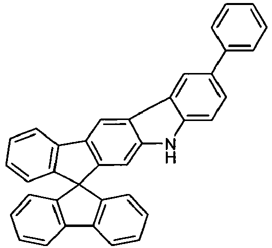 | 56% |
| 2e | 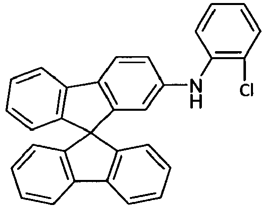 | 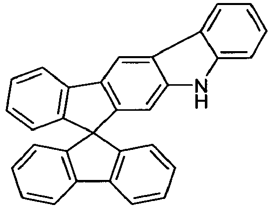 | 71% |
| 2f | 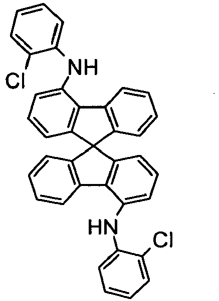 | 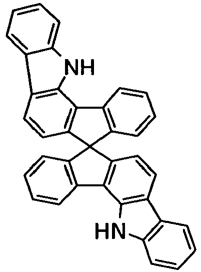 | 67% |
| 2g | 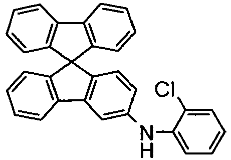 | 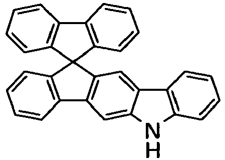 | 51% |
| 2h | 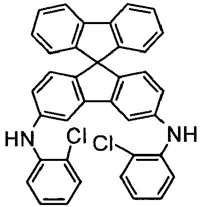 | 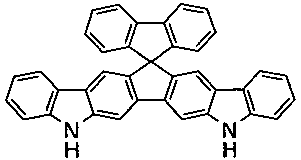 | 43% |
| 2i | 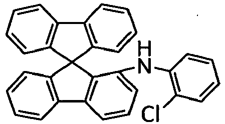 | 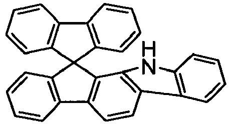 | 47% |

### Beispiel 3a: Nukleophile Substitution

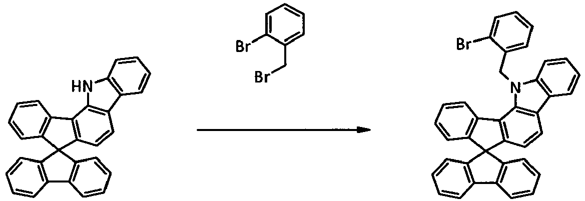

In einem 500 mL-Vierhalskolben werden 760 mg (19 mmol) Natriumhydrid in 50 mL THF vorgelegt. Über einen Tropftrichter werden 7.0 g (17 mmol) Spirocarbazol **2a,** gelöst in 200 mL THF, zugetropft und 1 h nachgerührt. Anschließend werden 4.3 g (17 mmol) 2-Brom-benzylbromid, gelöst in 100 mL THF zugetropft und 5 h bis zum vollständigen Umsatz bei Raumtemperatur gerührt. Das Reaktionsgemisch wird auf Eis gegeben und auf Raumtemperatur erwärmt. Der ausgefallene Feststoff wird filtriert und mit *n*-Heptan gewaschen und bei vermindertem Druck getrocknet. Das Produkt wirde als farbloser Feststoff erhalten. Ausbeute: 9.3 g(16 mmol, entspr. 94 % d. Th).

Analog können folgende Verbindungen erhalten werden.

| **Bsp**. | **Edukt 1** | **Edukt 2** | **Produkt** | **Ausbeute** |
|---|---|---|---|---|
| 3b | 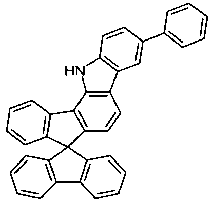 | 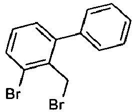 | 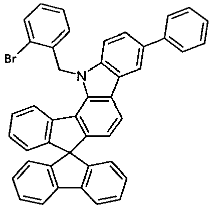 | 79% |
| | | 172976-02-2 | | |
| 3c | 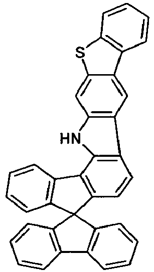 | 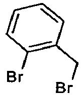 | 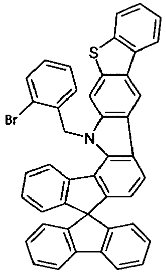 | 87 % |
| | | 3433-80-5 | | |
| 3d | 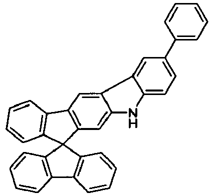 | 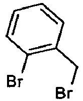 | 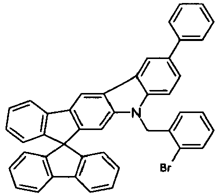 | 92% |
| | | 3433-80-5 | | |
| 3e | 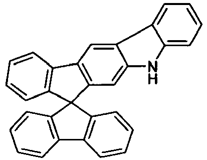 | 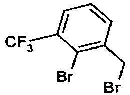 | 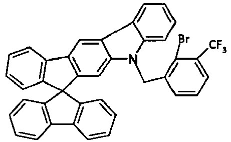 | 90% |
| | | 1214372-35-6 | | |
| 3f | 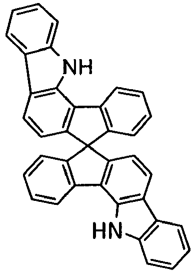 | 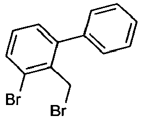 | 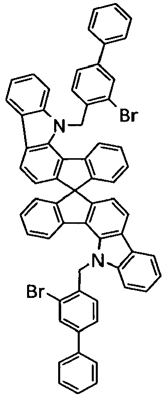 | 90% |
| | | 172976-02-2 2eq | | |
| 3g | 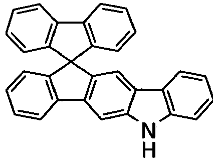 | 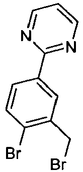 | 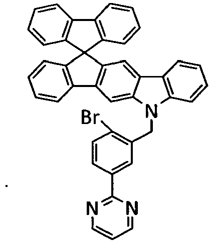 | 82% |
| | | 202805-71-8 | | |
| 3h | 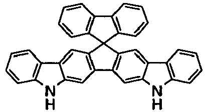 | 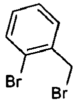 | 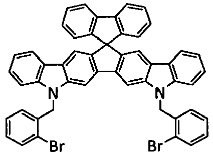 | 88% |
| | | 3433-80-5 2 eq | | |
| 3i | 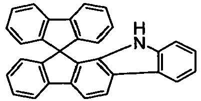 | 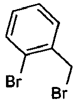 | 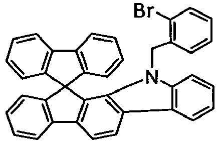 79% | |
| | | 3433-80-5 | | |

### Beispiel 4a: Cyclisierung

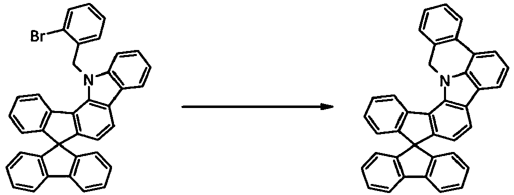

In einem 1-L-Vierhalskolben werden 29 g (50 mmol) **3a** und 9.90 g (101 mmol) Kaliumacetat in 500 mL DMF vorgelegt und 30 Minuten Argon durchgeleitet. Anschließend werden 1.75 g (1.51 mmol) Pd(PPh₃)₄ zugegeben und 16 h bis zur vollständigen Umsetzung unter Rückfluss erhitzt. Das Reaktionsgemisch wird auf Raumtemperatur abgekühlt und mit 400 mL Wasser hydrolysiert. Der ausgefallene Feststoff wird filtriert und mit Wasser gewaschen. Nach Trocknung unter vermindertem Druck wird das Produkt als grauer Feststoff erhalten. Ausbeute: 4.7 g (50.0 mmol; entspr. 99 %)

Analog können folgende Verbindungen erhalten werden:

| **Bsp**. | **Edukt 1** | **Produkt** | **Ausbeute** |
|---|---|---|---|
| 4b | 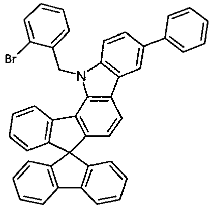 | 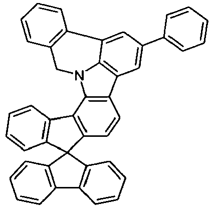 | 80% |
| 4c | 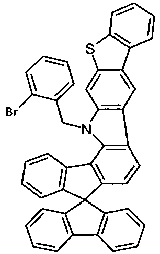 | 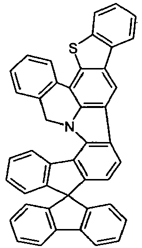 | 84% |
| 4d | 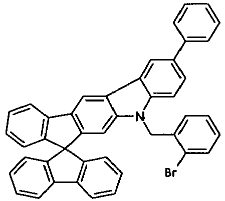 | 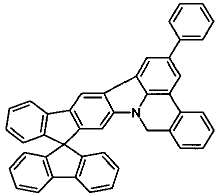 | 85% |
| 4e | 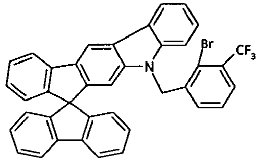 | 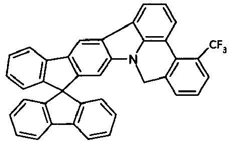 | 81 % |
| 4f | 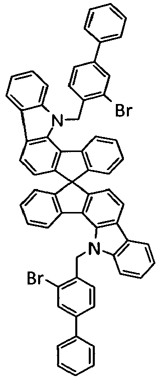 | 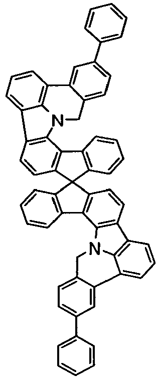 | 88 % |
| 4g | 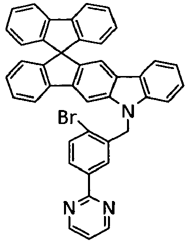 | 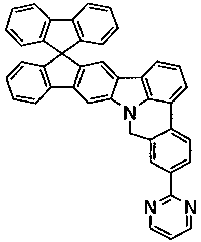 | 79% |
| 4h | 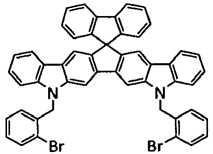 | 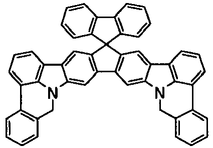 | 78% |
| 4i | 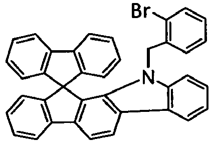 | 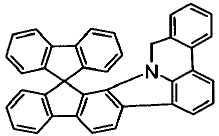 | 76% |

### Beispiel 5a: Oxidation

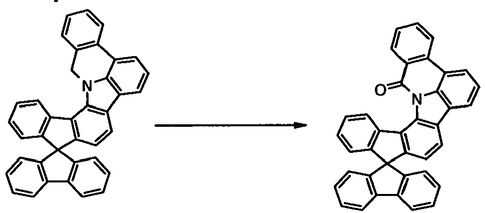

In einem 1-L-Vierhalskolben werden 23.5 g (47.6 mmol) **4a** in 500 mL Dichlormethan/Wasser (1:1) gelöst. Anschließend werden 17.5 g (47.6 mmol) Dibenzo-18-Krone-6 und portionsweise 91.0 g (576 mmol) Kaliumpermanganat zugegeben und 24 h bei Raumtemperatur und 4 Tage unter Rückfluss gerührt. Nach Abkühlen auf Raumtemperatur werden die Lösungsmittel unter vermindertem Druck entfernt. Der erhaltene Feststoff wird mit Toluol heiß extrahiert. Der dabei ausgefallene Feststoff wird filtriert und viermal mit 200 mL kaltem Acetonitril gewaschen. Anschließend wird bis zu einer HPLC-Reinheit >99.9 % mit Toluol heiß extrahiert und umkristallisiert. Nach zweimaligem Sublimieren (340 °C bei <10⁻⁴ bar) wird das Produkt in einer HPLC-Reinheit von >99.9 % als weißer Feststoff erhalten. Ausbeute: 3 g (7 mmol entspr. 15 %)

Analog können die folgenden Verbindungen erhalten werden:

| **Bsp**. | **Edukt 1** | **Produkt** | **Ausbeute** |
|---|---|---|---|
| 5b | 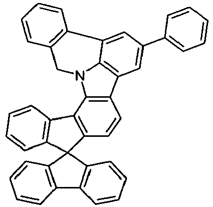 | 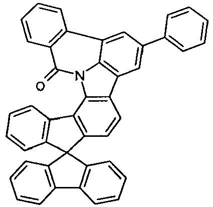 | 23% |
| 5c | 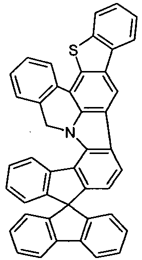 | 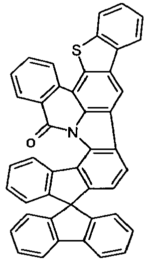 | 24 % |
| 5d | 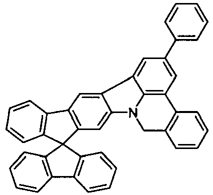 | 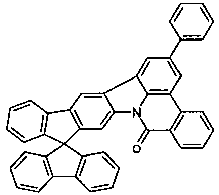 | 26 % |
| 5e | 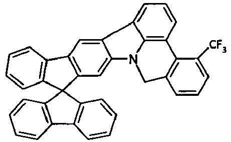 | 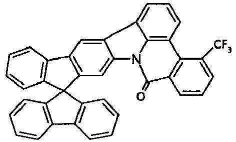 | 21 % |
| 5f | 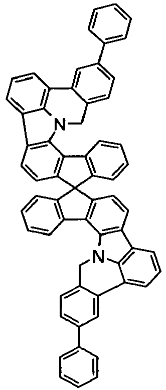 | 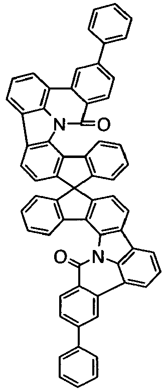 | 23 % |
| 5g | 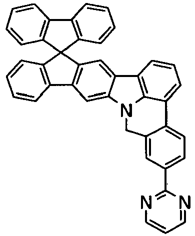 | 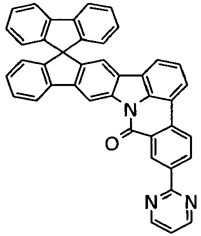 | 18% |
| 5h | 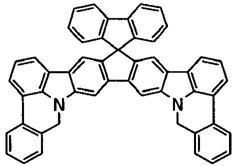 | 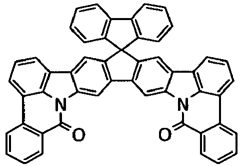 | 31 % |
| 5i | 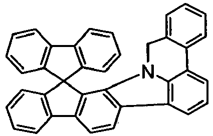 | 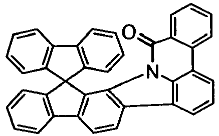 | 23% |

### Methode 2:

### Beispiel 6a: Nukleophile Substitution

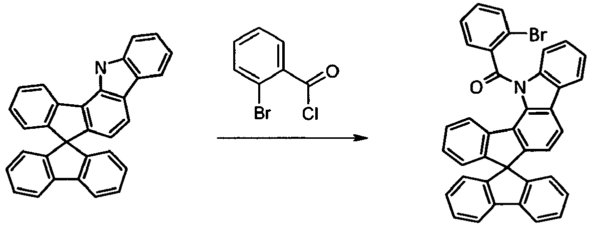

2.1 g (52.5 mmol) NaH 60%ig in Mineralöl werden in 500 mL THF unter Schutzatmosphäre gelöst. 33.8 g (50 mmol) Verbindung **2a** und 11.5 g (52.5 mmol) 15-Krone-5 gelöst in 200 ml THF werden zugegeben. Nach 1h bei Raumtemperatur wird eine Lösung von 12 g (55 mmol) 2-Brombenzoylchlorid in 250 mL THF zugetropft. Das Reaktionsgemisch wird 18 h bei Raumtemperatur gerührt. Nach dieser Zeit wird das Reaktionsgemisch auf Eis gegossen und dreimal mit Dichlormethan extrahiert. Die vereinigten organischen Phasen werden über Na₂SO₄ getrocknet und eingeengt. Der Rückstand wird mit Toluol heiß extrahiert und aus Toluol / n-Heptan umkristallisiert. Ausbeute: 17 g (29 mmol), 60%; Reinheit ca. 98 % nach ¹H-NMR.

Analog können folgende Verbindungen erhalten werden:

| **Bsp**. | **Edukt 1** | **Edukt 2** | **Produkt** | **Ausbeute** |
|---|---|---|---|---|
| 6b | 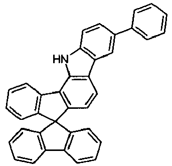 | 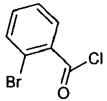 | 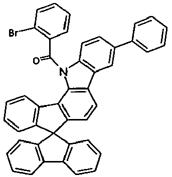 | 68% |
| | | 7154-66-7 | | |
| 6c | 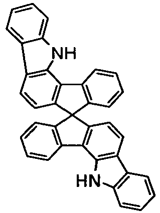 | 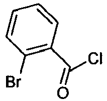 | 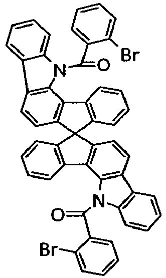 | 64% |
| | | 7154-66-7 | | |
| 6d | 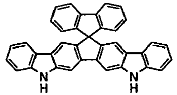 | 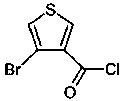 | 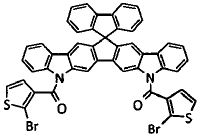 | 63% |
| | | 72899-51-5 | | |

### Beispiel 7a: Cyclisierung

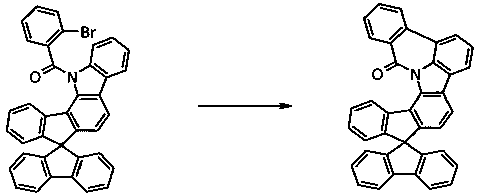

Unter Schutzgas werden 43 ml Tributylzinnhydrid (16 mmol) und 30 g (12-5 mmol) 1, 1'-Azobis(cyclohexan-1-carbonitril) in 600 ml Toluol über einen Zeitraum von 4 h zu einer siedende Lösung aus 7.4 g (12.5 mmol) Verbindung **5a** in 600 ml Toluol getropft. Anschließend wird 3 h unter Rückfluss erhitzt. Nach dieser Zeit wird das Reaktionsgemisch auf Eis gegossen und dreimal mit Dichlormethan extrahiert. Die vereinigten organischen Phasen werden über Na₂SO₄ getrocknet und eingeengt. Der Rückstand wird aus Toluol und aus Dichlormethan / *iso*-Propanol umkristallisiert und abschließend im Hochvakuum sublimiert, Reinheit beträgt 99.9%. Ausbeute: 4.2 g (8.2 mmol), 66%.

Analog können folgende Verbindungen erhalten werden:

| **Bsp**. | **Edukt 1** | **Produkt** | **Ausbeute** |
|---|---|---|---|
| 7b | 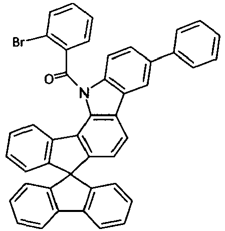 | 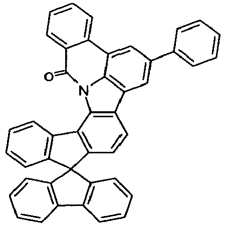 | 68% |
| 7c | 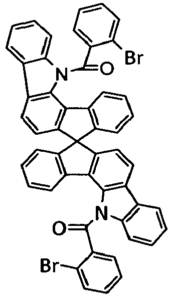 | 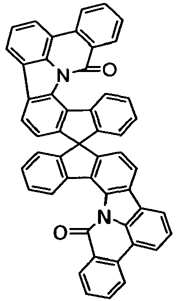 | 64% |
| 7d | 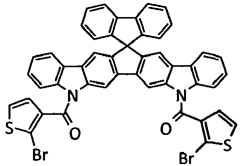 | 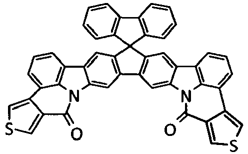 | 63% |

### Herstellung der OLEDs

In den folgenden Beispielen V1 bis E11 (siehe Tabellen 1 und 2) werden die Daten verschiedener OLEDs vorgestellt.

**Vorbehandlung für die Beispiele V1-E11:** Glasplättchen, die mit strukturiertem ITO (Indium Zinn Oxid) der Dicke 50 nm beschichtet sind, werden zur verbesserten Prozessierung mit 20 nm PEDOT:PSS beschichtet (Poly(3,4-ethylendioxythiophen)poly(styrolsulfonat), bezogen als CLEVIOS™ P VP Al 4083 von Heraeus Precious Metals GmbH Deutschland, aus wässriger Lösung aufgeschleudert). Diese beschichteten Glasplättchen bilden die Substrate, auf welche die OLEDs aufgebracht werden.

Die OLEDs haben prinzipiell folgenden Schichtaufbau: Substrat / Lochtransportschicht (HTL) / optionale Zwischenschicht (IL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs verwendeten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie 5e:IC2:TEG1 (45%:45%:10%) bedeutet hierbei, dass das Material aus Beispiel 5e in einem Volumenanteil von 45%, das Material IC2 in einem Volumenanteil von 45% und TEG1 in einem Volumenanteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m².

Die Daten der verschiedenen OLEDs sind in Tabelle 2 zusammengefasst. Die Beispiele V1- V2 sind Vergleichsbeispiele gemäß dem Stand der Technik, die Beispiele E1-E11 zeigen Daten von erfindungsgemäßen OLEDs.

Im Folgenden werden einige der Beispiele näher erläutert, um die Vorteile der erfindungsgemäßen OLEDs zu verdeutlichen.

### Verwendung von erfindungsgemäßen Mischungen in der Emissionsschicht phosphoreszenter OLEDs

Die erfindungsgemäßen Materialien ergeben bei Einsatz als Matrixmaterialien in phosphoreszierenden OLEDs wesentliche Verbesserungen der Spannung und der Leistungseffizienz gegenüber dem Stand der Technik. Durch Einsatz der erfindungsgemäßen Verbindung 5d in Kombination mit dem grün emittierenden Dotanden TEG1 lässt sich eine signifikante Verbesserung der Spannung und der Leistungseffizienz gegenüber dem Stand der Technik (V1, V2) beobachten (Beispiel E1).

**Tabelle 1: Aufbau der OLEDs**

| Bsp | HTL Dicke | IL Dicke | EBL Dicke | EML Dicke | HBL Dicke | ETL Dicke | EIL Dicke |
|---|---|---|---|---|---|---|---|
| V1 | SpA1 70nm | HATCN 5nm | SpMA1 90nm | SdT1:TEG1 (90%:10%) 40nm | --- | ST2:LiQ (50%:50%) 30nm | --- |
| V2 | SpA1 70nm | HATCN 5nm | SpMA1 90nm | SdT2:TEG1 (90%:10%) 40nm | --- | ST2:LiQ (50%:50%) 30nm | --- |
| E1 | SpA1 70nm | HATCN 5nm | SpMA1 90nm | 5d:TEG1 (90%:10%) 40nm | --- | ST2:LiQ (50%:50%) 30nm | --- |
| E2 | SpA1 90nm | HATCN 5nm | SpMA1 130nm | 5a:TER1 (92%:8%) 40nm | --- | ST2:LiQ (50%:50%) 40nm | --- |
| E3 | SpA1 90nm | HATCN 5nm | SpMA1 130nm | 5b:TER1 (92%:8%) 40nm | --- | ST2:LiQ (50%:50%) 40nm | --- |
| E4 | SpA1 70nm | HATCN 5nm | SpMA1 90nm | IC1:TEG1 (90%:10%) 30nm | --- | 5c:ST2 (50%:50%) 40nm | LiQ 3nm |
| E5 | SpA1 70nm | HATCN 5nm | SpMA1 90nm | 5e:IC2:TEG1 (45%:45%:10%) 40nm | --- | ST2:LiQ (50%:50%) 30nm | --- |
| E6 | SpA1 70nm | HATCN 5nm | SpMA1 90nm | 5f:TEG1 (90%:10%) 40nm | --- | ST2:LiQ (50%:50%) 30 nm | --- |
| E7 | SpA1 70nm | HATCN 5nm | SpMA1 90nm | 5d:TEG1 (90%:10%) 40nm | --- | ST2:LiQ (50%:50%) 30nm | --- |
| E8 | SpA1 70nm | HATCN 5nm | SpMA1 90nm | IC1:TEG1 (90%:10%) 30nm | --- | 5h:ST1 (50%:50%) 40nm | LiQ 3nm |
| E9 | SpA1 70nm | HATCN 5nm | SpMA1 90nm | IC1:TEG1 (90%:10%) 30nm | 5i 10nm | ST2:LiQ (50%:50%) 30nm | --- |
| E10 | SpA1 70nm | HATCN 5nm | SpMA1 90nm | IC1:TEG1 (90%:10%) 30nm | --- | 7c:ST2 (50%:50%) 40nm | LiQ 3nm |
| E11 | SpA1 70nm | HATCN 5nm | SpMA1 90nm | 7d:IC2:TEG1 (45%:45%:10%) 40nm | --- | ST2:LiQ (50%:50%) 30nm | --- |

**Tabelle 2: Daten der OLEDs**

| Bsp. | U1000 (V) | SE1000 (cd/A) | LE1000 (Im/W) | EQE 1000 | CIE x/y bei 1000 cd/m² |
|---|---|---|---|---|---|
| V1 | 5.3 | 46 | 27 | 12.9% | 0.33/0.62 |
| V2 | 3.8 | 52 | 43 | 14.5% | 0.33/0.62 |
| E1 | 3.4 | 54 | 50 | 14.7% | 0.34/0.63 |
| E2 | 5.4 | 13 | 8 | 13.1% | 0.66/0.34 |
| E3 | 4.6 | 13 | 9 | 12.7% | 0.67/0.33 |
| E4 | 3.5 | 59 | 53 | 16.2% | 0.34/0.63 |
| E5 | 3.4 | 60 | 55 | 16.6% | 0.33/0.62 |
| E6 | 4.2 | 47 | 35 | 13.2% | 0.37/0.60 |
| E7 | 3.3 | 57 | 54 | 15.2% | 0.33/0.63 |
| E8 | 3.4 | 60 | 55 | 16.4% | 0.34/0.63 |
| E9 | 3.8 | 60 | 50 | 16.0% | 0.33/0.63 |
| E10 | 3.5 | 59 | 53 | 15.9% | 0.34/0.62 |
| E11 | 3.4 | 58 | 54 | 15.5% | 0.34/0.63 |

**Tabelle 3: Strukturformeln der Materialien für die OLEDs**

| | |
|---|---|
| 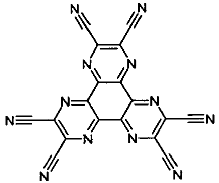 | 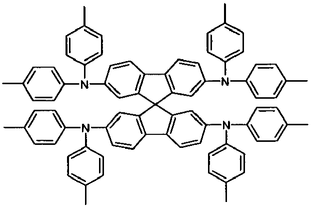 |
| HATCN | SpA1 |
| 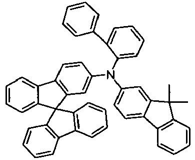 | 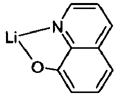 |
| SpMA1 | LiQ |
| 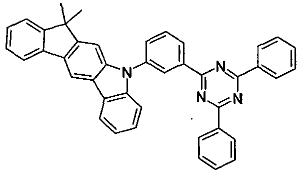 | 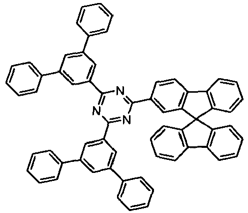 |
| IC2 | ST2 |
| 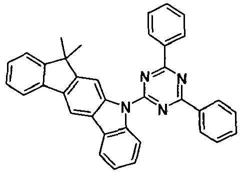 | 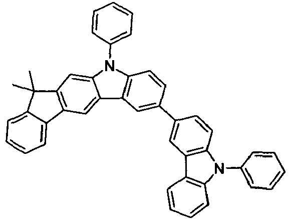 |
| IC1 | IC3 |
| 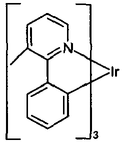 | 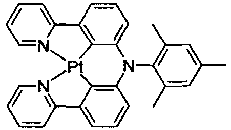 |
| TEG1 | TER1 |
| 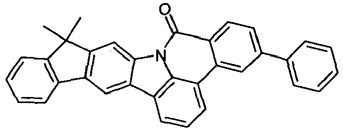 | 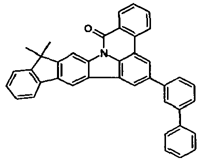 |
| SdT1 | SdT2 |
| 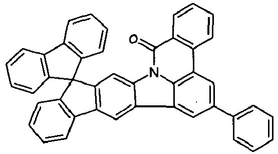 | 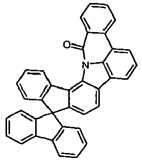 |
| 5d | 5a |
| 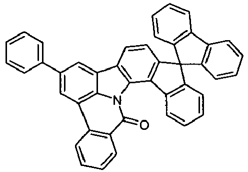 | 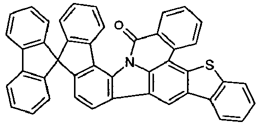 |
| 5b | 5c |
| 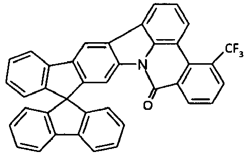 | 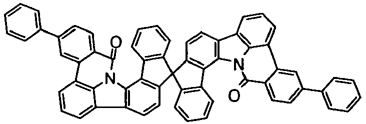 |
| 5e | 5f |
| 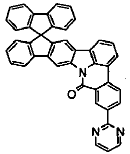 | 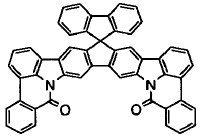 |
| 5g | 5h |
| 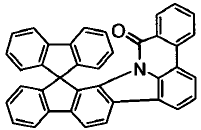 | 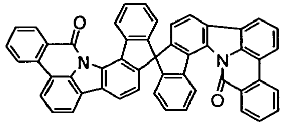 |
| 5i | 7c |
| 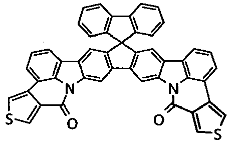 | |
| 7d | |

## Patentansprüche

1. Verbindung der Formel (1), 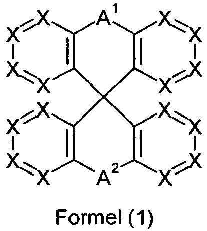 wobei für die verwendeten Symbole gilt:
X ist bei jedem Auftreten gleich oder verschieden CR oder N oder zwei benachbarte Gruppen X stehen zusammen für O, S oder NR, so dass sich ein Fünfring ergibt, oder zwei benachbarte Gruppen X stehen zusammen für eine Gruppe der folgenden Formel (2), (3) oder (4),
mit der Maßgabe, dass die Verbindung der Formel (1) mindestens eine Gruppe der Formel (2) aufweist, 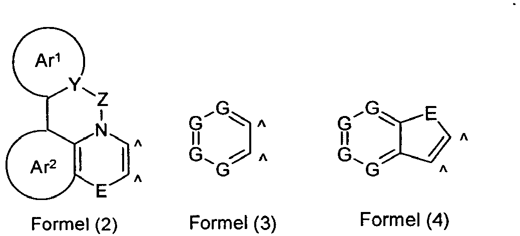 wobei ^ die Positionen andeutet, an denen die Gruppe der Formel (2) bzw. (3) bzw. (4) an die Verbindung der Formel (1) ankondensiert;
A¹, A² ist gleich oder verschieden bei jedem Auftreten eine Einfachbindung, CR₂, NR, O, S oder C=O;
Z ist gleich oder verschieden bei jedem Auftreten C=O, C=S, CR₂, BR, SiR₂, P(=O)R, SO oder SO₂;
Y ist C, wenn Ar¹ eine 6-Ring-Aryl- oder Heteroarylgruppe darstellt, bzw. ist C oder N, wenn Ar¹ eine 5-Ring-Heteroarylgruppe darstellt;
E ist gleich oder verschieden bei jedem Auftreten eine Einfachbindung, CR₂, NR, O, S oder C=O, mit der Maßgabe, dass E in Formel (4) keine Einfachbindung darstellt;
Ar¹ ist gleich oder verschieden bei jedem Auftreten zusammen mit der Gruppe Y und dem explizit dargestellten Kohlenstoffatom ein aromatisches oder heteroaromatisches Ringsystem mit 5 bis 24 aromatischen Ringatomen, welches durch einen oder mehrere Reste R substituiert sein kann;
Ar² ist gleich oder verschieden bei jedem Auftreten zusammen mit den drei explizit dargestellten Kohlenstoffatomen ein aromatisches oder heteroaromatisches Ringsystem mit 5 bis 24 aromatischen Ringatomen, welches durch einen oder mehrere Reste R substituiert sein kann;
G ist gleich oder verschieden bei jedem Auftreten CR oder N;
R ist bei jedem Auftreten gleich oder verschieden ausgewählt aus der Gruppe bestehend aus H, D, F, Cl, Br, I, CN, NO₂, N(Ar³)₂, N(R¹)₂, C(=O)Ar³, C(=O)R¹, P(=O)(Ar³)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R¹ substituiert sein kann, wobei eine oder mehrere nicht-benachbarte CH₂-Gruppen durch R¹C=CR¹, C≡C, Si(R¹)₂, C=O, C=NR¹, P(=O)(R¹), SO, SO₂, NR¹, O, S oder CONR¹ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R¹ substituiert sein kann, einer Aryloxy- oder Heteroaryloxygruppe mit 5 bis 40 aromatischen Ringatomen, die mit einem oder mehreren Resten R¹ substituiert sein kann, oder einer Aralkyl- oder Heteroaralkylgruppe mit 5 bis 40 aromatischen Ringatomen, die mit einem oder mehreren Resten R¹ substituiert sein kann, wobei optional zwei oder mehr benachbarte Substituenten R ein monocyclisches oder polycyclisches, aliphatisches, aromatisches oder heteroaromatisches Ringsystem bilden können, das mit einem oder mehreren Resten R¹ substituiert sein kann;
Ar³ ist bei jedem Auftreten gleich oder verschieden ein aromatisches oder heteroaromatisches Ringsystem mit 5-30 aromatischen Ringatomen, das mit einem oder mehreren nicht-aromatischen Resten R¹ substituiert sein kann; dabei können zwei Reste Ar³, welche an dasselbe N-Atom oder P-Atom binden, auch durch eine Einfachbindung oder eine Brücke, ausgewählt aus N(R¹), C(R¹)₂ oder O, miteinander verbrückt sein;
R¹ ist bei jedem Auftreten gleich oder verschieden ausgewählt aus der Gruppe bestehend aus H, D, F, Cl, Br, I, CN, NO₂, N(R²)₂, C(=O)R², P(=O)(R²)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R² substituiert sein kann, wobei eine oder mehrere nicht-benachbarte CH₂-Gruppen durch R²C=CR², C≡C, Si(R²)₂, C=O, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R² substituiert sein kann, einer Aryloxy- oder Heteroaryloxygruppe mit 5 bis 40 aromatischen Ringatomen, die mit einem oder mehreren Resten R² substituiert sein kann, oder einer Aralkyl- oder Heteroaralkylgruppe mit 5 bis 40 aromatischen Ringatomen, die mit einem oder mehreren Resten R² substituiert sein kann, wobei optional zwei oder mehr benachbarte Substituenten R¹ ein monocyclisches oder polycyclisches, aliphatisches, aromatisches oder heteroaromatisches Ringsystem bilden können, das mit einem oder mehreren Resten R² substituiert sein kann;
R² ist ausgewählt aus der Gruppe bestehend aus H, D, F, CN, einem aliphatischem Kohlenwasserstoffrest mit 1 bis 20 C-Atomen, einem aromatischem oder heteroaromatischem Ringsystem mit 5 bis 30 aromatischen Ringatomen, wobei zwei oder mehr benachbarte Substituenten R² miteinander ein mono- oder polycyclisches, aliphatisches, aromatisches oder heteroaromatisches Ringsystem bilden können.

2. Verbindung nach Anspruch 1, **dadurch gekennzeichnet, dass** A¹ und A² für Einfachbindungen stehen.

3. Verbindung nach Anspruch 1 oder 2, **dadurch gekennzeichnet, dass** sie genau eine oder zwei Gruppen der Formel (2) enthält.

4. Verbindung nach einem oder mehreren der Ansprüche 1 bis 3, ausgwählt aus den Verbindungen der Formel (5) bis (10), 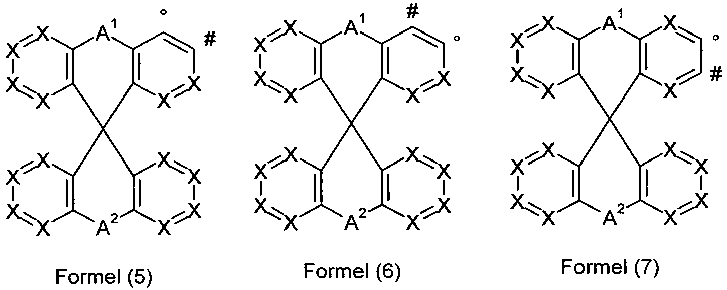 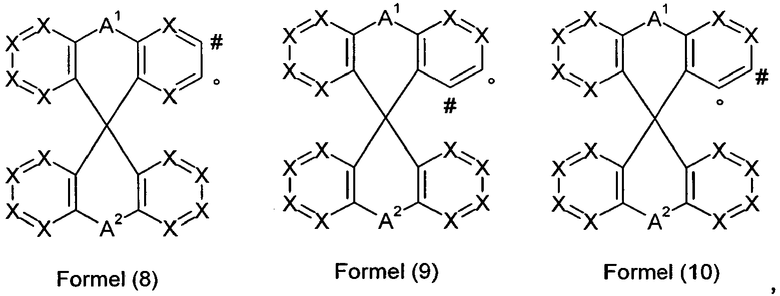 wobei ^{∘} die Verknüpfung mit dem Stickstoffatom der Gruppe der Formel (2) darstellt, # die Verknüpfung mit der Gruppe E der Gruppe der Formel (2) darstellt und die weiteren verwendeten Symbole die in Anspruch 1 genannten Bedeutungen aufweisen.

5. Verbindung nach einem oder mehreren der Ansprüche 1 bis 4, ausgewählt aus den Verbindungen der Formeln (5a) bis (10a), 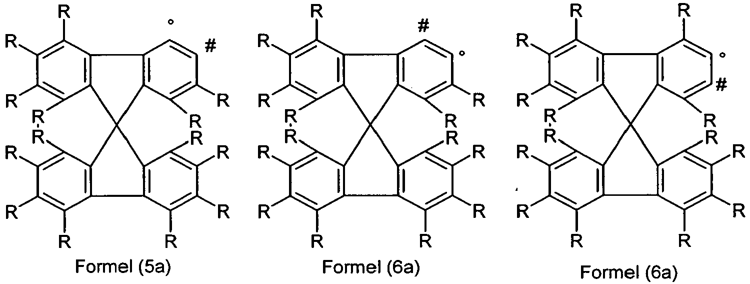 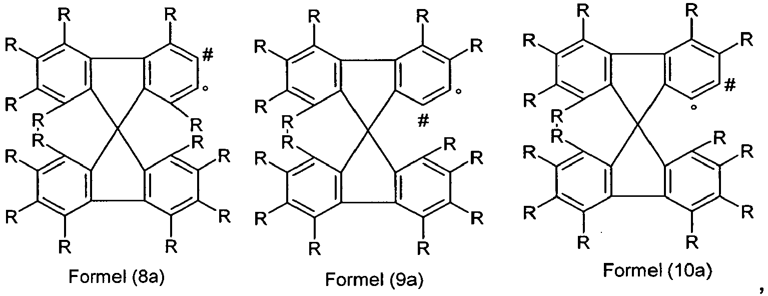 wobei die verwendeten Symbole die in Anspruch 1 und 4 genannten Bedeutungen aufweisen.

6. Verbindung nach einem oder mehreren der Ansprüche 1 bis 5, ausgewählt aus Verbindungen der Formeln (5b) bis (10b), 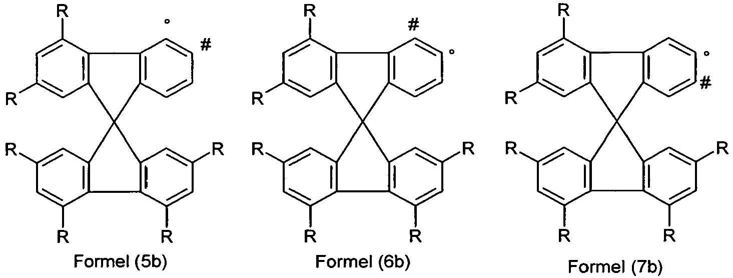 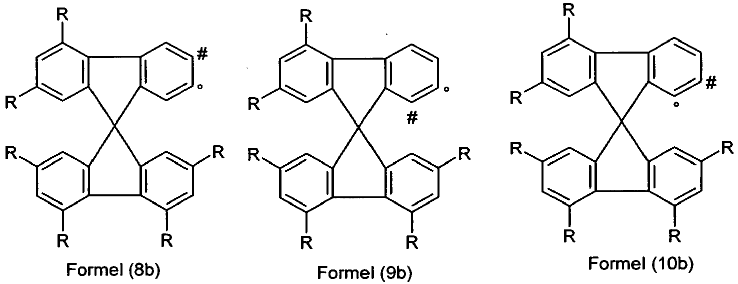 wobei die verwendeten Symbole die in Anspruch 1 und 4 genannten Bedeutungen aufweisen.

7. Verbindung nach einem oder mehreren der Ansprüche 1 bis 6, **dadurch gekennzeichnet, dass** in der Gruppe der Formel (2) Z gleich oder verschieden für C=O oder C=S steht und dass E für eine Einfachbindung, CR₂, C=O oder NR steht.

8. Verbindung nach einem oder mehreren der Ansprüche 1 bis 7, **dadurch gekennzeichnet, dass** Ar¹ für eine Gruppe der Formel (13), (14), (15), (16) oder (17) steht, 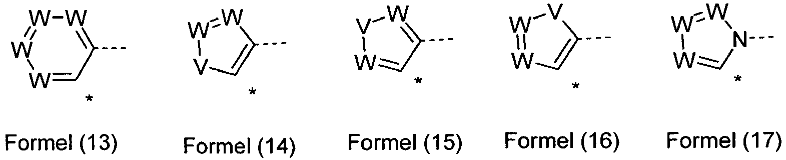 wobei die gestrichelte Bindung die Verknüpfung mit Z andeutet und * die Position der Verknüpfung mit Ar² andeutet;
und dass Ar² für eine Gruppe der Formel (20), (21) oder (22) steht, 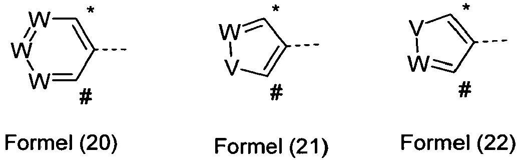 wobei die gestrichelte Bindung die Verknüpfung mit N andeutet, # die Position der Verknüpfung mit E andeutet und * die Verknüpfung mit Ar¹ andeutet;
und weiterhin gilt:
W ist gleich oder verschieden bei jedem Auftreten CR oder N; oder zwei benachbarte Gruppen W stehen für eine Gruppe der folgenden Formel (18) oder (19), 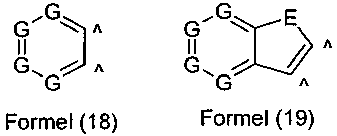 wobei E die in Anspruch 1 genannte Bedeutung aufweist, jedoch keine Einfachbindung darstellt, G gleich oder verschieden bei jedem Auftreten für CR oder N steht und ^ die entsprechenden benachbarten Gruppen W in der Formel (13) bis (17) andeuten;
V ist NR, O oder S.

9. Verbindung nach einem oder mehreren der Ansprüche 1 bis 8, **dadurch gekennzeichnet, dass** die Gruppe der Formel (2) ausgewählt ist aus den Gruppen der Formeln (23) bis (29), 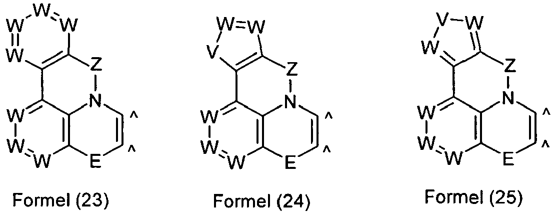 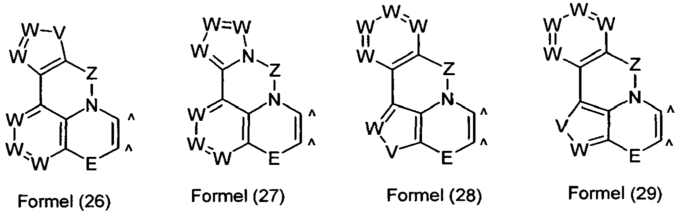 wobei ^ die Position der Verknüpfung in Formel (1) kennzeichnet und die weiteren verwendeten Symbole die in Anspruch 1 und 8 genannten Bedeutungen aufweisen.

10. Verbindung nach einem oder mehreren der Ansprüche 1 bis 9, **dadurch gekennzeichnet, dass** die Gruppe der Formel (2) ausgewählt ist aus den Gruppen der Formeln (23a) bis (29a), 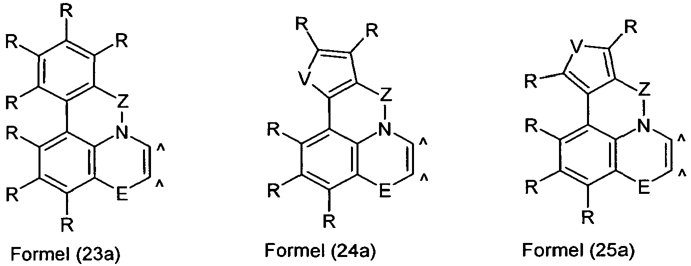 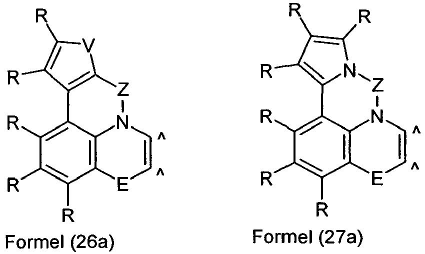 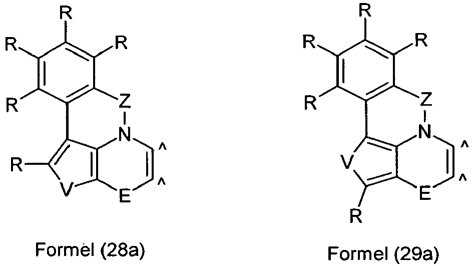 wobei die verwendeten Symbole die in Anspruch 1 und 8 genannten Bedeutungen aufweisen.

11. Verbindung nach einem oder mehreren der Ansprüche 1 bis 10, ausgewählt aus den Verbindungen der Formeln (5c) bis (10c), 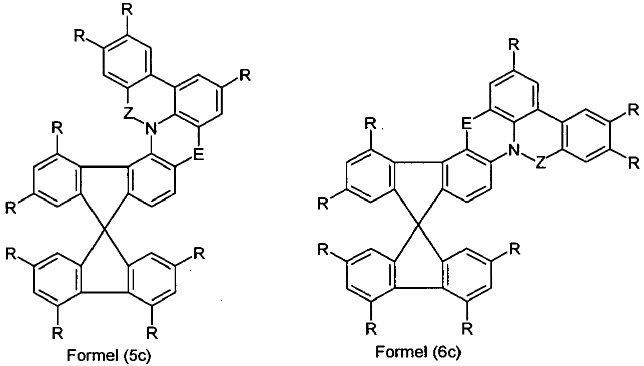 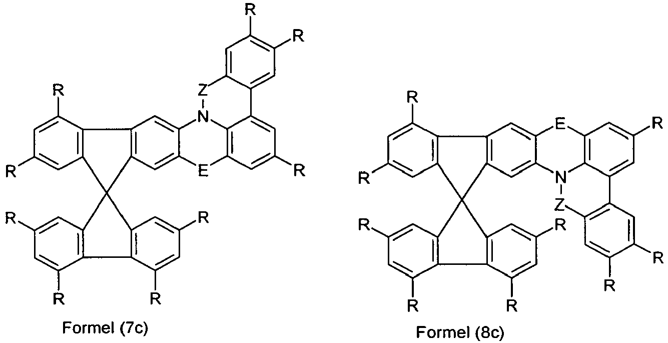 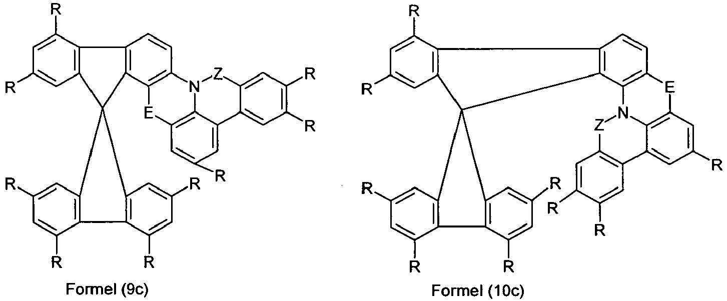 wobei die verwendeten Symbole die in Anspruch 1 genannten Bedeutungen aufweisen.

12. Verbindung nach einem oder mehreren der Ansprüche 1 bis 11, **dadurch gekennzeichnet, dass** alle Reste R für H stehen oder dass ein oder zwei Reste R für ein aromatisches oder heteroaromatisches Ringsystem mit 6 bis 24 aromatischen Ringatomen stehen, das jeweils mit einem oder mehreren Resten R¹ substituiert sein kann, und die anderen Reste R für H stehen.

13. Oligomer, Polymer oder Dendrimer enthaltend eine oder mehrere der Verbindungen nach einem oder mehreren der Ansprüche 1 bis 12, wobei statt einem oder mehreren Resten R eine oder mehrere Bindungen der Verbindung zum Polymer, Oligomer oder Dendrimer vorhanden sind.

14. Formulierung, enthaltend mindestens eine Verbindung nach einem oder mehreren der Ansprüche 1 bis 12 und/oder mindestens ein Oligomer, Polymer oder Dedrimer nach Anspruch 13 und mindestens eine weitere Verbindung, insbesondere ein Lösemittel oder eine Mischung mehrerer Lösemittel.

15. Verwendung einer Verbindung nach einem oder mehreren der Ansprüche 1 bis 12 und/oder eines Oligomers, Polymers oder Dedrimers nach Anspruch 13 in einer elektronischen Vorrichtung.

16. Elektronische Vorrichtung enthaltend mindestens eine Verbindung nach einem oder mehreren der Ansprüche 1 bis 12 und/oder mindestens ein Oligomer, Polymer oder Dedrimer nach Anspruch 13.

17. Elektronische Vorrichtung nach Anspruch 16, **dadurch gekennzeichnet, dass** es sich um eine organische Elektrolumineszenzvorrichtung handelt und die Verbindung nach einem oder mehreren der Ansprüche 1 bis 12 in einer emittierenden Schicht als Matrixmaterial für fluoreszierende oder phosphoreszierende Emitter und/oder in einer Lochblockierschicht und/oder in einer Elektronentransportschicht eingesetzt wird.

## Claims

1. Compound of the formula (1), 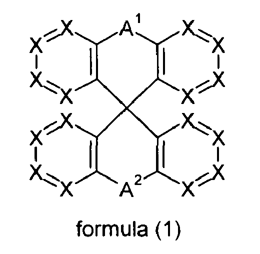 where the following applies to the symbols used:
X is on each occurrence, identically or differently, CR or N or two adjacent groups X together stand for O, S or NR, giving a five-membered ring, or two adjacent groups X together stand for a group of the following formula (2), (3) or (4),
with the proviso that the compound of the formula (1) contains at least one group of the formula (2), 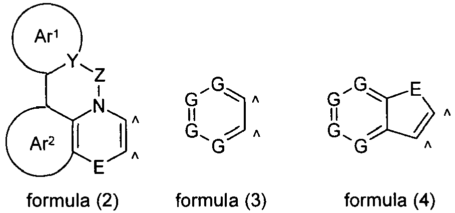 where ^ indicates the positions at which the group of the formula (2) or (3) or (4) condenses onto the compound of the formula (1);
A¹, A², identically or differently on each occurrence, are a single bond, CR₂, NR, O, S or C=O;
Z, identically or differently on each occurrence, is C=O, C=S, CR₂, BR, SiR₂, P(=O)R, SO or SO₂;
Y is C if Ar¹ represents a 6-membered aryl or heteroaryl group, or is C or N if Ar¹ represents a 5-membered heteroaryl group;
E, identically or differently on each occurrence, is a single bond, CR₂, NR, O, S or C=O, with the proviso that E in formula (4) does not represent a single bond;
Ar¹, identically or differently on each occurrence, together with the group Y and the carbon atom explicitly depicted, is an aromatic or heteroaromatic ring system having 5 to 24 aromatic ring atoms, which may be substituted by one or more radicals R;
Ar², identically or differently on each occurrence, together with the three carbon atoms explicitly depicted, is an aromatic or heteroaromatic ring system having 5 to 24 aromatic ring atoms, which may be substituted by one or more radicals R;
G, identically or differently on each occurrence, is CR or N;
R is on each occurrence, identically or differently, selected from the group consisting of H, D, F, Cl, Br, I, CN, NO₂, N(Ar³)₂, N(R¹)₂, C(=O)Ar³, C(=O)R¹, P(=O)(Ar³)₂, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, which may in each case be substituted by one or more radicals R¹, where one or more non-adjacent CH₂ groups may be replaced by R¹C=CR¹, C≡C, Si(R¹)₂, C=O, C=NR¹, P(=O)(R¹), SO, SO₂, NR¹, O, S or CONR¹ and where one or more H atoms may be replaced by D, F, Cl, Br, I, CN or NO₂, an aromatic or heteroaromatic ring system having 5 to 40 aromatic ring atoms, which may in each case be substituted by one or more radicals R¹, an aryloxy- or heteroaryloxy group having 5 to 40 aromatic ring atoms, which may be substituted by one or more radicals R¹, or an aralkyl or heteroaralkyl group having 5 to 40 aromatic ring atoms, which may be substituted by one or more radicals R¹, where two or more adjacent substituents R may optionally form a monocyclic or polycyclic, aliphatic, aromatic or heteroaromatic ring system, which may be substituted by one or more radicals R¹;
Ar³ is on each occurrence, identically or differently, an aromatic or heteroaromatic ring system having 5-30 aromatic ring atoms, which may be substituted by one or more non-aromatic radicals R¹; two radicals Ar³ here which are bonded to the same N atom or P atom may also be bridged to one another by a single bond or a bridge selected from N(R¹), C(R¹)₂ or O;
R¹ is on each occurrence, identically or differently, selected from the group consisting of H, D, F, Cl, Br, I, CN, NO₂, N(R²)₂, C(=O)R², P(=O)(R²)₂, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, which may in each case be substituted by one or more radicals R², where one or more non-adjacent CH₂ groups may be replaced by R²C=CR², C≡C, Si(R²)₂, C=O, C=NR², P(=O)(R²), SO, SO₂, NR², O, S or CONR² and where one or more H atoms may be replaced by D, F, Cl, Br, I, CN or NO₂, an aromatic or heteroaromatic ring system having 5 to 40 aromatic ring atoms, which may in each case be substituted by one or more radicals R², an aryloxy or heteroaryloxy group having 5 to 40 aromatic ring atoms, which may be substituted by one or more radicals R², or an aralkyl or heteroaralkyl group having 5 to 40 aromatic ring atoms, which may be substituted by one or more radicals R², where two or more adjacent substituents R¹ may optionally form a monocyclic or polycyclic, aliphatic, aromatic or heteroaromatic ring system, which may be substituted by one or more radicals R²;
R² is selected from the group consisting of H, D, F, CN, an aliphatic hydrocarbon radical having 1 to 20 C atoms, an aromatic or heteroaromatic ring system having 5 to 30 aromatic ring atoms, where two or more adjacent substituents R² may form a mono- or polycyclic, aliphatic, aromatic or heteroaromatic ring system with one another.

2. Compound according to Claim1, **characterised in that** A¹ and A² stand for single bonds.

3. Compound according to Claim1 or 2, **characterised in that** it contains precisely one or two groups of the formula (2).

4. Compound according to one or more of Claims 1 to 3, selected from the compounds of the formulae (5) to (10), 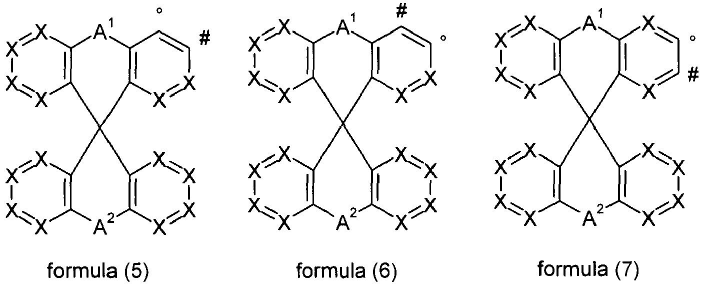 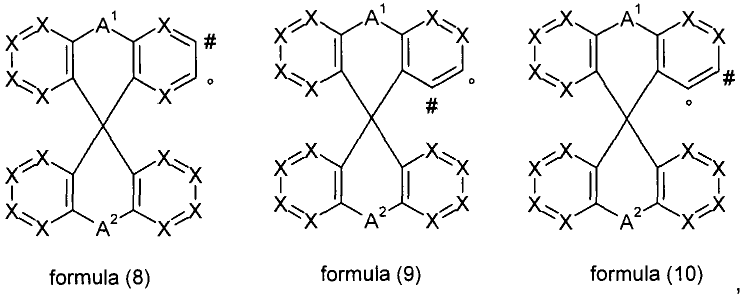 where ^{∘} represents the link to the nitrogen atom of the group of the formula (2), # represents the link to the group E of the group of the formula (2) and the other symbols used have the meanings given in Claim 1.

5. Compound according to one or more of Claims 1 to 4, selected from the compounds of the formulae (5a) to (10a), 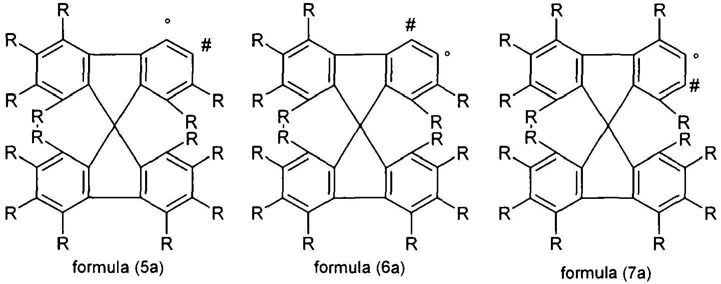 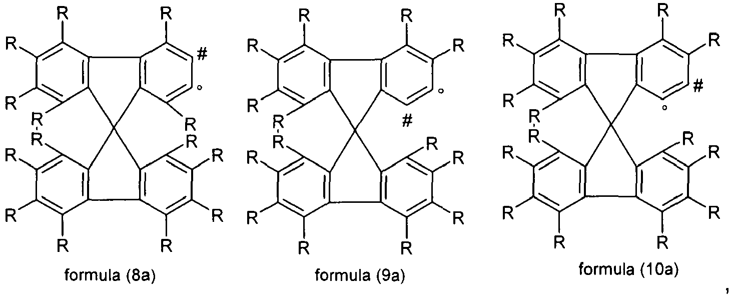 where the symbols used have the meanings given in Claims 1 and 4.

6. Compound according to one or more of Claims 1 to 5, selected from compounds of the formulae (5b) to (10b), 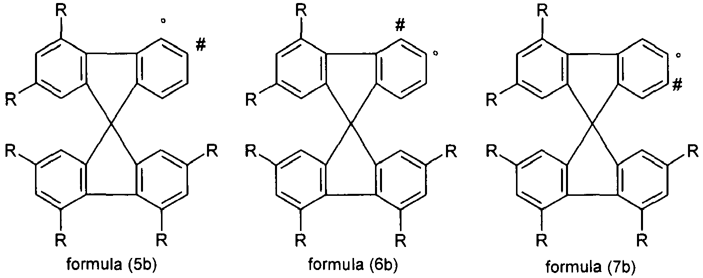 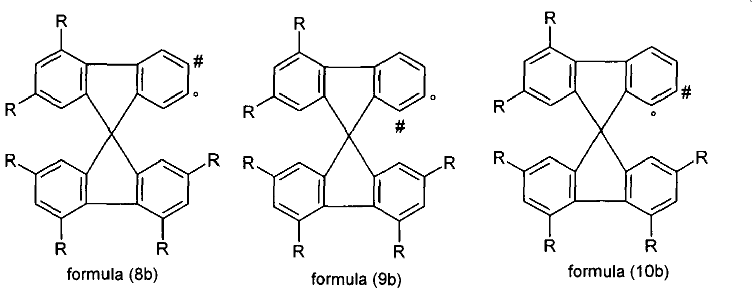 where the symbols used have the meanings given in Claims 1 and 4.

7. Compound according to one or more of Claims 1 to 6, **characterised in that** in Z in the group of the formula (2) stands, identically or differently, for C=O or C=S and **in that** E stands for a single bond, CR₂, C=O or NR.

8. Compound according to one or more of Claims 1 to 7, **characterised in that** Ar¹ stands for a group of the formula (13), (14), (15), (16) or (17), 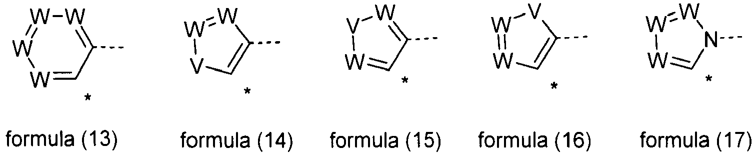 where the dashed bond indicates the link to Z and * indicates the position of the link to Ar²;
and **in that** Ar² stands for a group of the formula (20), (21) or (22), 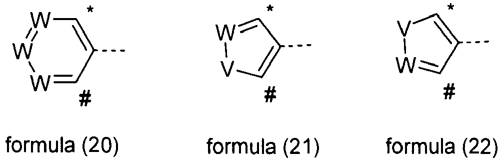 where the dashed bond indicates the link to N, # indicates the position of the link to E and * indicates the link to Ar¹;
and furthermore:
W is, identically or differently on each occurrence, CR or N; or two adjacent groups W stand for a group of the following formula (18) or (19), 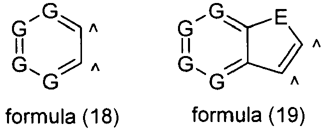 where E has the meaning given in Claim 1, but does not represent a single bond, G stands, identically or differently on each occurrence, for CR or N and ^ indicates the corresponding adjacent groups W in the formulae (13) to (17);
V is NR, O or S.

9. Compound according to one or more of Claims 1 to 8, **characterised in that** the group of the formula (2) is selected from the groups of the formulae (23) to (29), 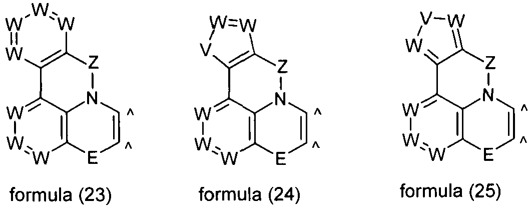 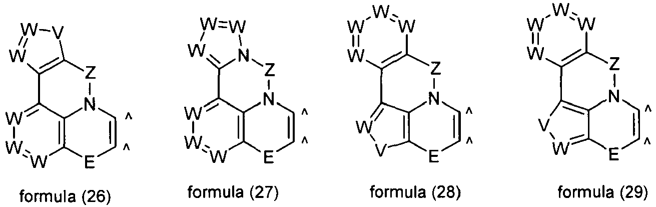 where ^ denotes the position of the link in formula (1) and the other symbols used have the meanings given in Claims 1 and 8.

10. Compound according to one or more of Claims 1 to 9, **characterised in that** the group of the formula (2) is selected from the groups of the formulae (23a) to (29a), 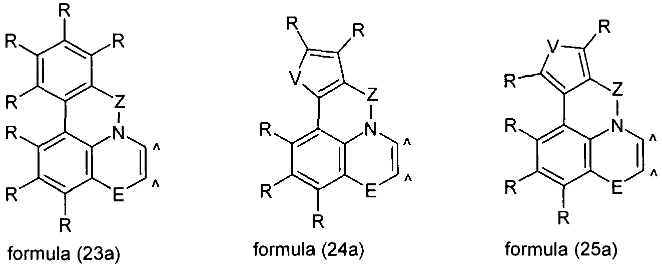 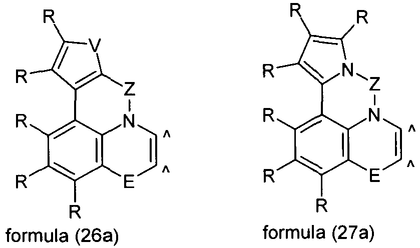 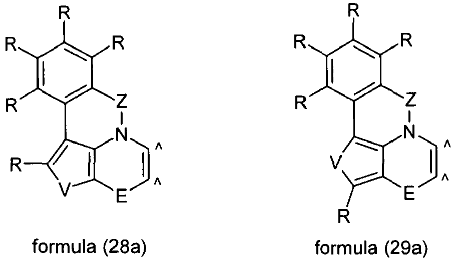 where the symbols used have the meanings given in Claims 1 and 8.

11. Compound according to one or more of Claims 1 to 10, selected from the compounds of the formulae (5c) to (10c), 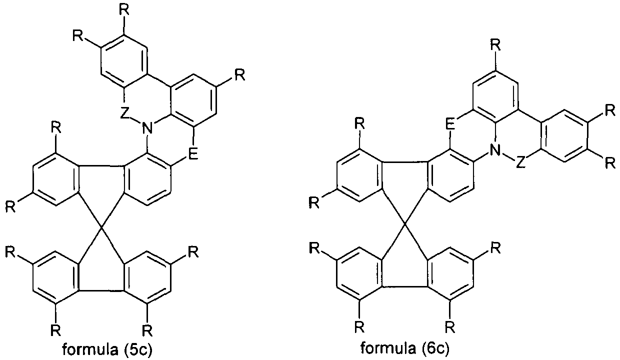 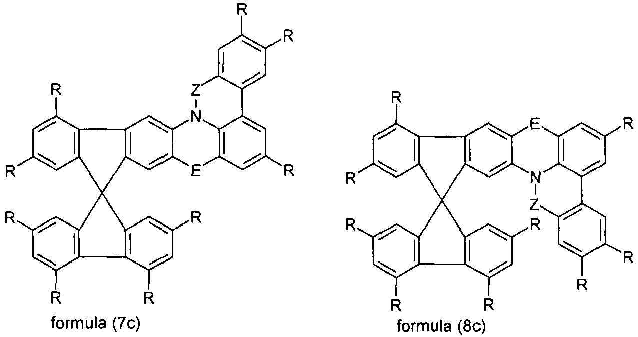 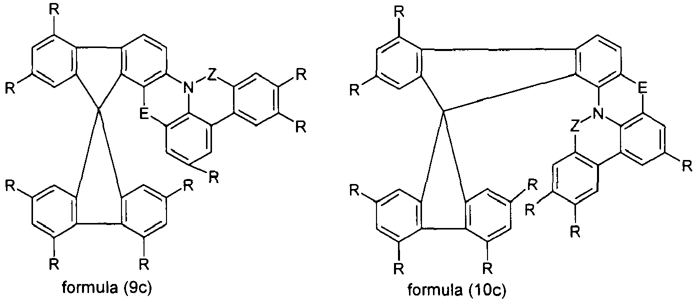 where the symbols used have the meanings given in Claim 1.

12. Compound according to one or more of Claims 1 to 11, **characterised in that** all radicals R stand for H or **in that** one or two radicals R stand for an aromatic or heteroaromatic ring system having 6 to 24 aromatic ring atoms, which may in each case be substituted by one or more radicals R¹, and the other radicals R stand for H.

13. Oligomer, polymer or dendrimer containing one or more of the compounds according to one or more of Claims 1 to 12, where one or more bonds from the compound to the polymer, oligomer or dendrimer are present instead of one or more radicals R.

14. Formulation comprising at least one compound according to one or more of Claims 1 to 12 and/or at least one oligomer, polymer or dendrimers according to Claim 13 and at least one further compound, in particular a solvent or a mixture of a plurality of solvents.

15. Use of a compound according to one or more of Claims 1 to 12 and/or an oligomer, polymer or dendrimer according to Claim 13 in an electronic device.

16. Electronic device containing at least one compound according to one or more of Claims 1 to 12 and/or at least one oligomer, polymer or dendrimer according to Claim 13.

17. Electronic device according to Claim 16, **characterised in that** it is an organic electroluminescent device and the compound according to one or more of Claims 1 to 12 is employed in an emitting layer as matrix material for fluorescent or phosphorescent emitters and/or in a holeblocking layer and/or in an electron-transport layer.

## Revendications

1. Composé de la formule (1) : 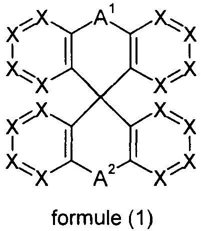 dans laquelle ce qui suit s'applique aux symboles utilisés :
X est pour chaque occurrence, de manière identique ou différente, CR ou N ou deux groupes X adjacents représentent ensemble O, S ou NR, ce qui donne un cycle à cinq éléments, ou deux groupes X adjacents représentent ensemble un groupe de la formule (2), (3) ou (4) qui suit :
étant entendu que le composé de la formule (1) contient au moins un groupe de la formule (2) : 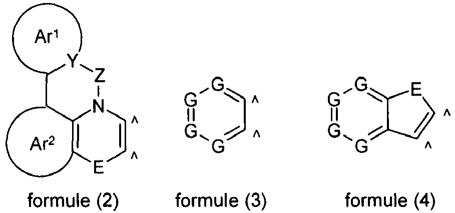 formule dans lesquelles ^ représente les positions au niveau desquelles le groupe de la formule (2) ou (3) ou (4) est condensé sur le composé de la formule (1) ;
A¹, A² sont, de manière identique ou différente pour chaque occurrence, une liaison simple, CR₂, NR, O, S ou C=O ;
Z est, de manière identique ou différente pour chaque occurrence, C=O, C=S, CR₂, BR, SiR₂, P(=O)R, SO ou SO₂ ;
Y est C si Ar¹ représente un groupe aryle ou hétéroaryle à 6 éléments, ou est C ou N si Ar¹ représente un groupe hétéroaryle à 5 éléments ;
E est, de manière identique ou différente pour chaque occurrence, une liaison simple, CR₂, NR, O, S ou C=O, étant entendu que E dans la formule (4) ne représente pas une liaison simple ;
Ar¹ est, de manière identique ou différente pour chaque occurrence, en association avec le groupe Y et l'atome de carbone représenté de manière explicite, un système de cycle aromatique ou hétéroaromatique qui comporte 5 à 24 atomes de cycle aromatique, lequel peut être substitué par un radical ou plusieurs radicaux R ;
Ar² est, de manière identique ou différente pour chaque occurrence, en association avec le groupe Y et les trois atomes de carbone représentés de manière explicite, un système de cycle aromatique ou hétéroaromatique qui comporte 5 à 24 atomes de cycle aromatique, lequel peut être substitué par un radical ou plusieurs radicaux R ;
G est, de manière identique ou différente pour chaque occurrence, CR ou N ;
R est, de manière identique ou différente pour chaque occurrence, sélectionné parmi le groupe qui est constitué par H, D, F, Cl, Br, I, CN, NO₂, N(Ar³)₂, N(R¹)₂, C(=O)Ar³, C(=O)R¹, P(=O)(Ar³)₂, un groupe alkyle, alcoxy ou thioalkyle en chaîne droite qui comporte 1 à 40 atome(s) de C ou un groupe alkyle, alcoxy ou thioalkyle ramifié ou cyclique qui comporte 3 à 40 atomes de C ou un groupe alkényle ou alkynyle qui comporte 2 à 40 atomes de C, lequel peut dans chaque cas être substitué par un radical ou plusieurs radicaux R¹, où un ou plusieurs groupe(s) CH₂ non adjacents peut/peuvent être remplacé(s) par R¹C=CR¹, C≡C, Si(R¹)₂, C=O, C=NR¹, P(=O)(R¹), SO, SO₂, NR¹, O, S ou CONR¹ et où un ou plusieurs atome(s) de H peut/peuvent être remplacé(s) par D, F, Cl, Br, I, CN ou NO₂, un système de cycle aromatique ou hétéroaromatique qui comporte 5 à 40 atomes de cycle aromatique, lequel peut dans chaque cas être substitué par un radical ou plusieurs radicaux R¹, un groupe aryloxy ou hétéroaryloxy qui comporte 5 à 40 atomes de cycle aromatique, lequel peut être substitué par un radical ou plusieurs radicaux R¹, ou un groupe aralkyle ou hétéroaralkyle qui comporte 5 à 40 atomes de cycle aromatique, lequel peut être substitué par un radical ou plusieurs radicaux R¹, où deux substituants R adjacents ou plus peuvent en option former un système de cycle aromatique ou hétéroaromatique aliphatique monocyclique ou polycyclique, lequel peut être substitué par un radical ou plusieurs radicaux R¹ ;
Ar³ est pour chaque occurrence, de manière identique ou différente, un système de cycle aromatique ou hétéroaromatique qui comporte 5 à 30 atomes de cycle aromatique, lequel peut être substitué par un radical ou plusieurs radicaux R¹ non aromatique(s) ; deux radicaux Ar³ ici qui sont liés au même atome de N ou au même atome de P peuvent également être pontés l'un à l'autre au moyen d'une liaison simple ou d'un pont qui est sélectionné parmi N(R¹), C(R¹)₂ ou O ;
R¹ est pour chaque occurrence, de manière identique ou différente, sélectionné parmi le groupe qui est constitué par H, D, F, Cl, Br, I, CN, NO₂, N(R²)₂, C(=O)R², P(=O)(R²)₂, un groupe alkyle, alcoxy ou thioalkyle en chaîne droite qui comporte 1 à 40 atome(s) de C ou un groupe alkyle, alcoxy ou thioalkyle ramifié ou cyclique qui comporte 3 à 40 atomes de C ou un groupe alkényle ou alkynyle qui comporte 2 à 40 atomes de C, lequel peut dans chaque cas être substitué par un radical ou plusieurs radicaux R², où un ou plusieurs groupe(s) CH₂ non adjacents peut/peuvent être remplacé(s) par R²C=CR², C≡C, Si(R²)₂, C=O, C=NR², P(=O)(R²), SO, SO₂, NR², O, S ou CONR² et où un ou plusieurs atome(s) de H peut/peuvent être remplacé(s) par D, F, Cl, Br, I, CN ou NO₂, un système de cycle aromatique ou hétéroaromatique qui comporte 5 à 40 atomes de cycle aromatique, lequel peut dans chaque cas être substitué par un radical ou plusieurs radicaux R², un groupe aryloxy ou hétéroaryloxy qui comporte 5 à 40 atomes de cycle aromatique, lequel peut être substitué par un radical ou plusieurs radicaux R², ou un groupe aralkyle ou hétéroaralkyle qui comporte 5 à 40 atomes de cycle aromatique, lequel peut être substitué par un radical ou plusieurs radicaux R², où deux substituants R¹ adjacents ou plus peuvent en option former un système de cycle aromatique ou hétéroaromatique aliphatique monocyclique ou polycyclique, lequel peut être substitué par un radical ou plusieurs radicaux R² ;
R² est sélectionné parmi le groupe qui est constitué par H, D, F, CN, un radical hydrocarbone aliphatique qui comporte 1 à 20 atome(s) de C, un système de cycle aromatique ou hétéroaromatique qui comporte 5 à 30 atomes de cycle aromatique, où deux substituants R² adjacents ou plus peuvent former un système de cycle aromatique ou hétéroaromatique aliphatique monocyclique ou polycyclique l'un avec l'autre ou les uns avec les autres.

2. Composé selon la revendication 1, **caractérisé en ce que** A¹ et A² représentent des liaisons simples.

3. Composé selon la revendication 1 ou 2, **caractérisé en ce qu'**il contient précisément un ou deux groupe(s) de la formule (2).

4. Composé selon une ou plusieurs des revendications 1 à 3, sélectionné parmi les composés des formules (5) à (10) : 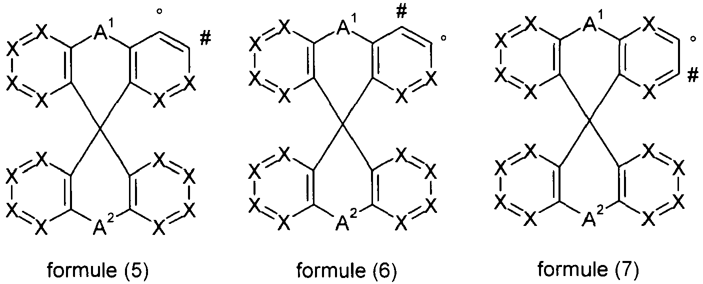 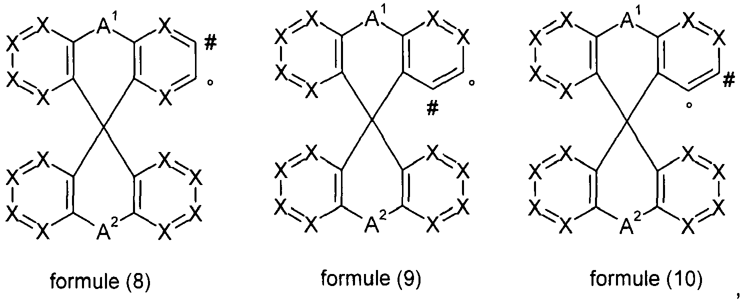 formules dans lesquelles ^{∘} représente la liaison sur l'atome d'azote du groupe de la formule (2), # représente la liaison sur le groupe E du groupe de la formule (2) et les autres symboles utilisés présentent les significations qui ont été données selon la revendication 1.

5. Composé selon une ou plusieurs des revendications 1 à 4, sélectionné parmi les composés des formule (5a) à (10a) : 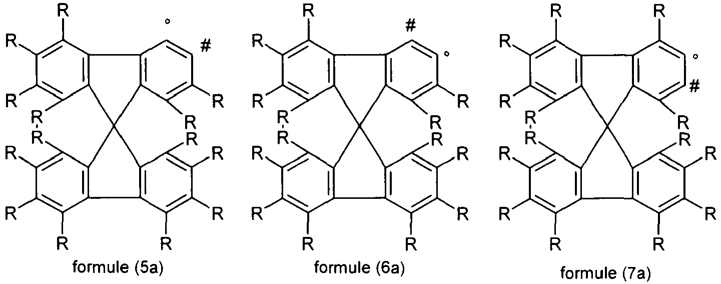 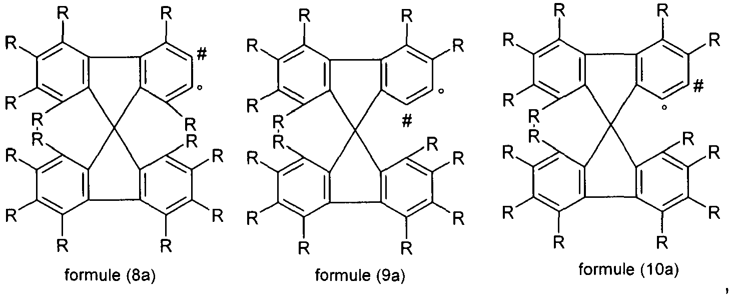 formules dans lesquelles les symboles utilisés présentent les significations qui ont été données selon les revendications 1 et 4.

6. Composé selon une ou plusieurs des revendications 1 à 5, sélectionné parmi les composés des formules (5b) à (10b) : 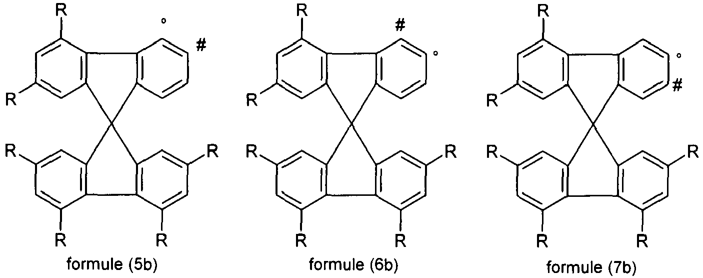 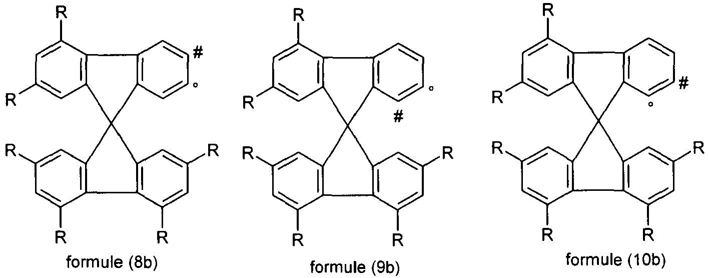 formules dans lesquelles les symboles utilisés présentent les significations qui ont été données selon les revendications 1 et 4.

7. Composé selon une ou plusieurs des revendications 1 à 6, **caractérisé en ce que** Z dans le groupe de la formule (2) représente, de manière identique ou différente, C=O ou C=S et **en ce que** E représente une liaison simple, CR₂, C=O ou NR.

8. Composé selon une ou plusieurs des revendications 1 à 7, **caractérisé en ce que** Ar¹ représente un groupe de la formule (13), (14), (15), (16) ou (17) : 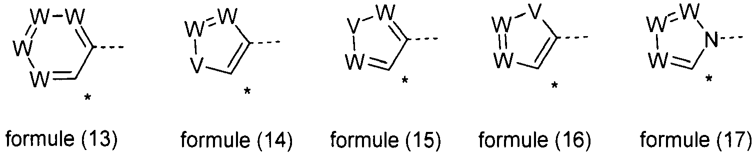 formules dans lesquelles la liaison en pointillés indique la liaison sur Z et * indique la position de la liaison sur Ar² ;
et **en ce que** Ar² représente un groupe de la formule (20), (21) ou (22) : 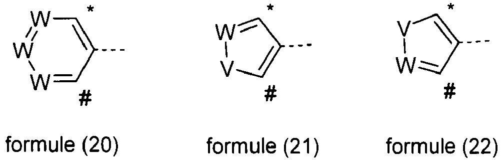 formules dans lesquelles la liaison en pointillés indique la liaison sur N, # indique la position de la liaison sur E et * indique la liaison sur Ar¹ ;
et en outre :
W est, de manière identique ou différente pour chaque occurrence, CR ou N ; ou deux groupes W adjacents représentent un groupe de la formule (18) ou (19) qui suit : 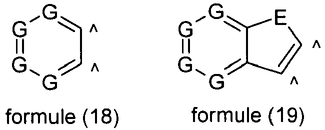 formules dans lesquelles E présente la signification qui a été donnée selon la revendication 1, mais ne représente pas une liaison simple, G représente, de manière identique ou différente pour chaque occurrence, CR ou N et ^ indique les groupes W adjacents correspondants dans les formules (13) à (17) ;
V est NR, O ou S.

9. Composé selon une ou plusieurs des revendications 1 à 8, **caractérisé en ce que** le groupe de la formule (2) est sélectionné parmi les groupes des formules (23) à (29) : 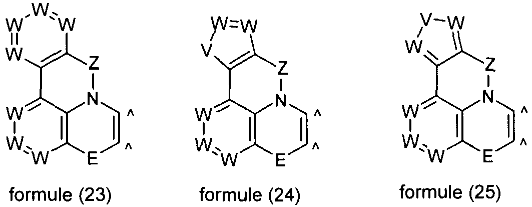 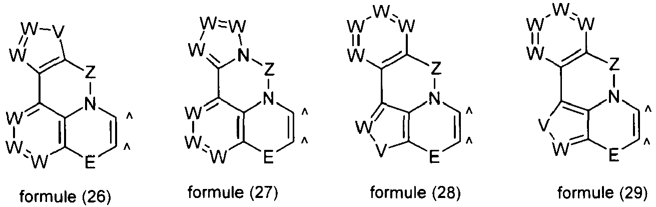 formules dans lesquelles ^ représente la position de la liaison dans la formule (1) et les autres symboles utilisés présentent les significations qui ont été données selon les revendications 1 et 8.

10. Composé selon une ou plusieurs des revendications 1 à 9, **caractérisé en ce que** le groupe de la formule (2) est sélectionné parmi les groupes des formules (23a) à (29a) : 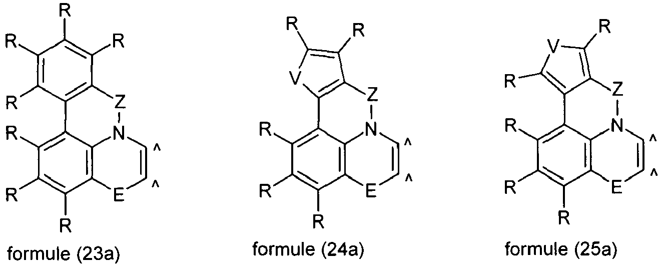 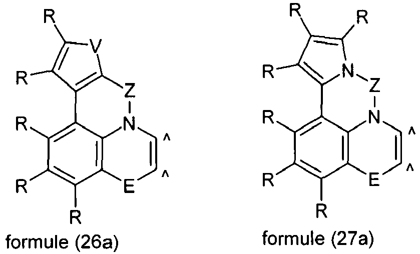 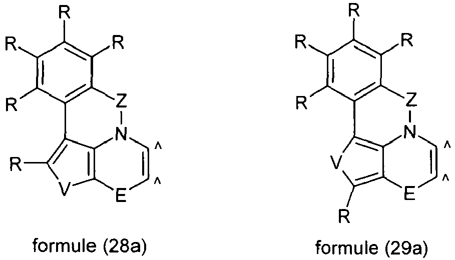 formules dans lesquelles les symboles utilisés présentent les significations qui ont été données selon les revendications 1 et 8.

11. Composé selon une ou plusieurs des revendications 1 à 10, sélectionné parmi les composés des formules (5c) à (10c) : 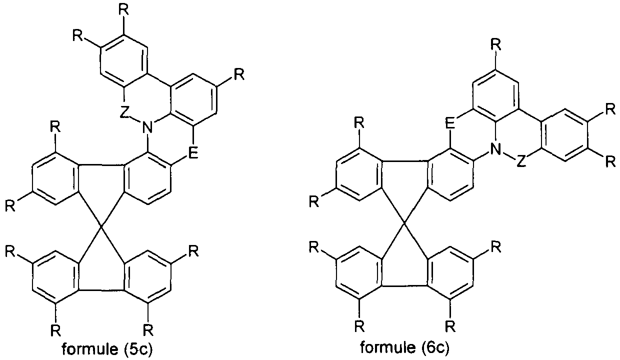 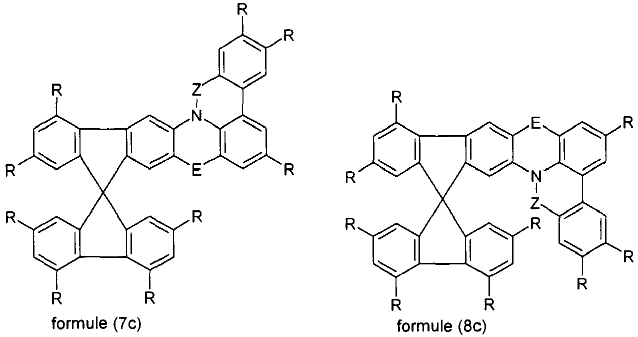 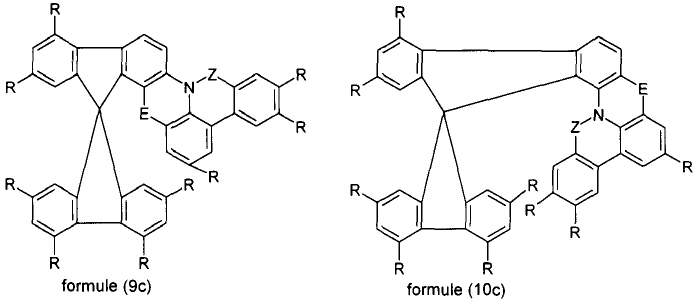 formules dans lesquelles les symboles utilisés présentent les significations qui ont été données selon la revendication 1.

12. Composé selon une ou plusieurs des revendications 1 à 11, **caractérisé en ce que** tous les radicaux R représentent H ou **en ce qu'**un radical ou deux radicaux R représente(nt) un système de cycle aromatique ou hétéroaromatique qui comporte 6 à 24 atomes de cycle aromatique, lequel peut dans chaque cas être substitué par un radical ou plusieurs radicaux R¹, et les autres radicaux R représentent H.

13. Oligomère, polymère ou dendrimère contenant un ou plusieurs des composés selon une ou plusieurs des revendications 1 à 12, dans lequel une ou plusieurs liaison(s) depuis le composé sur le polymère, l'oligomère ou le dendrimère est/sont présente(s) en lieu et place d'un radical ou de plusieurs radicaux R.

14. Formulation comprenant au moins un composé selon une ou plusieurs des revendications 1 à 12 et/ou au moins un oligomère, un polymère ou un dendrimère selon la revendication 13 et au moins un autre composé, en particulier un solvant ou un mélange d'une pluralité de solvants.

15. Utilisation d'un composé selon une ou plusieurs des revendications 1 à 12 et/ou d'un oligomère, d'un polymère ou d'un dendrimère selon la revendication 13 dans un dispositif électronique.

16. Dispositif électronique contenant au moins un composé selon une ou plusieurs des revendications 1 à 12 et/ou au moins un oligomère, un polymère ou un dendrimère selon la revendication 13.

17. Dispositif électronique selon la revendication 16, **caractérisé en ce qu'**il s'agit d'un dispositif électroluminescent organique et le composé selon une ou plusieurs des revendications 1 à 12 est utilisé dans une couche d'émission en tant que matériau de matrice pour des émetteurs fluorescents ou phosphorescents et/ou dans une couche de blocage de trous et/ou dans une couche de transport d'électrons.
